# EUROPEAN PATENT SPECIFICATION

(11) **EP 1 582 176 B1**
(45) Date of publication and mention of the grant of the patent: **09.04.2008**
(21) Application number: 05103868.5
(22) Date of filing: 09.02.2001
(51) Int. Cl.: A61F 2/00, A61F 2/26

(54) **Mechanical impotence treatment apparatus**
Mechanische Vorrichtung zur Impotenzbehandlung
Appareil mécanique de traitement de l'impuissance

(30) Priority: 10.02.2000 US 181552 P
(43) Date of publication of application: 05.10.2005
(62) Divisional of application: 01902955.2
(73) Proprietor: Potencia Medical AG, 6341 Baar (CH)
(72) Inventor: Forsell, Peter, 6300, Zug (CH)
(74) Representative: Holmberg, Martin Tor

(56) References cited:
- WO-A-99/63907
- US-A- 4 419 985

## Description

The present invention relates to a male sexual impotence treatment apparatus, comprising an adjustable restriction device implantable in a male patient, who suffers from sexual impotence, for directly engaging a portion of the normal penile tissue or the prolongation thereof of the patient, and an operable adjustment device implantable in the patient for adjusting the restriction device to temporarily contract said portion of the normal penile tissue or the prolongation thereof to restrict the blood flow leaving the penis, when the patient desires to achieve erection. The closest prior art is WO 99630A, which defines the preamble of claim 1.

Male sexual impotence is a widespread problem. Many different solutions to this problem have been tried. A main solution currently practised and disclosed in for instance U.S. Patent Nos. 5437605 and 4841461 is to implant a hydraulic inflatable/contractible silicon prosthesis in the cavities of the corpora cavernosa of the patient's penis. In fluid connection with this prosthesis is a reservoir implanted in the scrotum. By manual pumping action the prosthesis is filled with fluid from the reservoir to effect errect condition or is emptied of fluid, which returns to the reservoir, to effect flaccid condition.

However, there are several more or less severe disadvantages of this main solution. Above all, the penis is more or less damaged by the operation and it is practically impossible to reverse the operation. Another disadvantage is that rather strong forces act against this implanted prosthesis resulting in a significant risk of the prosthesis being broken. A further disadvantage is that hard fibrosis created around the reservoir over time may cause malfunction of pumping components. Thus, the created fibrosis will sooner or later become a hard fibrotic layer which may make it difficult to pump the reservoir. Yet a further disadvantage is that the use of hydraulic fluid always entails a risk of fluid leaking from the prosthesis. Furthermore, it is a rather complicated task to manually pump the reservoir when erection is desired.

Another solution to achieve erection is to restrict the blood flow leaving the penis. For example, U.S. Patent No. 4829990 discloses two hydraulically operated inflatable cuffs wrapped around the respective crura. Again, a disadvantage of such a solution is that it entails a risk of hydraulic fluid leaking from the cuffs.

Another example of the solution to restrict the penile blood flow is found in U.S. Patent No. 4828544, which discloses an artificial fistula system surgically implanted and providing a primary fistula between the femoral artery and the femoral vein and a secondary fistula for leading blood from the the primary fistula to the penis. An inflatable balloon engages the primary fistula between the secondary fistula and the vein. The balloon is in fluid connection with a manually compressible reservoir implanted in the scrotum. Besides the risk of fluid leaking from the balloon, a further disadvantage of this latter example is that it requires delicate surgery.

A main disadvantage of the solution to restrict the penile blood flow is the fact that the venous blood vessel system is rather complex and it is difficult to restrict the veinplexa.

Yet another solution is to inject a substance in the vein system to achieve erection. However, injections are painful and complicated for the patient.

The prime object of the present invention is to provide a male sexual impotence treatment apparatus in which the risk of liquid leaking from implanted hydraulic components of the apparatus is substantially reduced or completely eliminated.

A further object of the invention is to provide a male sexual impotence treatment apparatus, which does not require surgical implantation of any fistula system.

Another object of the invention is to provide a male sexual impotence treatment apparatus, which does not require the use of an injection needle.

These objects are obtained by the invention as defined in claim 1.

Preferably, the adjustment device adjusts the restriction device in a non-invasive manner. Furthermore, the adjustment device may adjust the restriction device in a non-manual manner and/or in a non-magnetic manner, i.e., magnetic forces may not be involved when adjusting the restriction device.

Furthermore, as opposed to prior art impotence treatment devices the adjustment device of the invention preferably is non-manually operated, i.e. not operated by manual forces or manipulated by touching the skin of the patient, such as by manually compressing a fluid containing balloon implanted in the scrotum. Instead the apparatus of the invention may further comprise a powered operation device for operating the adjustment device.

In the various embodiments hereinafter described the restriction device generally forms an at least substantially closed loop. However, the restriction device may take a variety of different shapes, such as the shape of a square, rectangle or ellipse. The substantially closed loop could for example be totally flat, i.e. thin as seen in the radial direction. The shape of restriction device may also be changed during use, by rotation or movements of the restriction device in any direction.

A physical lumen, such as the corpus cavernosum, crura of the penile tissue or the prolongation thereof, is often easier to restrict by contracting at least two opposite or different side walls of the lumen against each other. The expression "penile tissue and the prolongation thereof" should be understood to mean the penile tissue extended inside the human body and following the pathway of the blood flow leaving the penis i.e. one or several exit veins from the penis, corpus cavernosum, crura or the prolongation thereof. Thus, the restriction device may be designed to perform such a contracting effect of the opposite walls also of a exit vein in the penile prolongation.

Alternatively, the restriction device may comprise an adjustable cuff, a clamp or a roller for bending the vein, corpus cavernosum, crura or the prolongation thereof to restrict the blood flow therein. Such a cuff, clamp or roller may also be utilised for squeezing the vein, corpus cavernosum, crura or the prolongation thereof against human material inside the body of the patient or against implanted structures of the apparatus.

Preferably, the restriction device comprises an elongated restriction member and forming means for forming the restriction member into at least a substantially closed loop around said portion of the tissue, wherein the loop defines a restriction opening, whereby the adjustment device adjusts the restriction member in the loop to change the size of the restriction opening.

The restriction device may be implanted in the base of the patient's penis or the prolongation thereof and preferably may engage the corpus cavernosum, crura or the prolongation thereof of the penis. However, there are several alternative positions of the restriction device that give more or less satisfactory restriction of the blood flow leaving the penis. Thus, as a first alternative the restriction member may extend around both corpora cavernosa or crura of the penis as a single unit. As a second alternative the restriction device may comprise two elongated restriction members extending around respective corpora cavernosa or crura of the patient. As a third alternative the elongated restriction member may encircle one of the exit veins from the penis. As a fourth alternative the restriction device may comprise several restriction members extending around respective exit veins from the penis.

The adjustment device may be incorporated in the restriction device as well as controlled by hydraulic means.

The restriction device may in all applicable embodiments take any shape.

The adjustment device is operable by a hydraulic operation device, which preferably is manually activatable. The hydraulic operation device may advantageously include hydraulic servo means to facilitate manual activation. As an alternative, the hydraulic device may be powered by an electric motor, which may be manually activatable or controlled by remote control means. The components of such a hydraulic operation device may be placed in association with the restriction device and/or be located at a suitable place in the abdomen or subcutaneously.

More specifically, a reservoir is provided containing a predetermined amount of fluid for supplying the hydraulic operation device with fluid. The reservoir defines a chamber for the predetermined amount of fluid and the hydraulic operation device changes the size of the chamber. The hydraulic operation device may comprise first and second wall portions of the reservoir, which are displaceable relative to each other to change the size of the chamber of the reservoir. The first and second wall portions of the reservoir may be designed to be displaceable relative to each other by manual manipulation thereof, preferably to permit manual pushing, pulling or rotation of any of the wall portions in one direction. Alternatively, the wall portions may be displaceable relative to each other by magnetic means (such as a permanent magnet and magnetic material reed switch, or other known or conventional magnetic devices), hydraulic means or electrical control means such as an electric motor. The magnetic means, hydraulic means, or electrical control means may all be activated by manual manipulation, preferably using a subcutaneously located manually manipulatable device. This control may be indirect, for example via a switch.

The hydraulic operation device may operate the adjustment device with fluid from the reservoir in response to a predetermined first displacement of the first wall portion of the reservoir relative to the second wall portion of the reservoir, to adjust the restriction device to release the tissue, and to operate the adjustment device with fluid from the reservoir in response to a predetermined second displacement of the first wall portion of the reservoir relative to the second wall portion of the reservoir, to adjust the restriction device to restrict the blood flow leaving the penis. In this embodiment, no pump is used, only the volume of the reservoir is varied. This is of great advantage compared to the solution described below when a pump is used to pump fluid between the reservoir and the adjustment device because there is no need for a non-return valve and it is still possible to have fluid going both to and from the reservoir.

As an alternative, the hydraulic operation device may comprise a pump for pumping fluid between the reservoir and the adjustment device. The pump may pump fluid both to and away from the adjustment device, or hydraulic means controlling the adjustment device. A mechanical manual solution is proposed in which it is possible to pump in both directions just by pushing an activating member in one direction. Another alternative is a pump pumping in only one direction and an adjustable valve to change the direction of fluid to either increase or decrease the amount of fluid in the reservoir. This valve may be manipulated manually, mechanically, electrically, magnetically, or hydraulically. Any kind of motor could of course be used for all the different operations as well as wireless remote solutions. The pump may comprise a first activation member for activating the pump to pump fluid from the reservoir to the adjustment device and a second activation member for activating the pump to pump fluid from the adjustment device to the reservoir. The activation members may be operable by manual manipulation, preferably to permit manual pushing, pulling or rotating thereof in one direction. Suitably, at least one of the activation members is adapted to operate when subjected to an external pressure exceeding a predetermined magnitude.

Alternatively, at least one of the first and second activating members may be operable by magnetic means, hydraulic means or electrical control means such as an electric motor. The magnetic means, hydraulic means, or electrical control means may all be activated by manual manipulating means preferably located subcutaneously. This activation may be indirect, for example via a switch.

Advantageously, especially when manual manipulation means are used, a servo means could be used. With servo means less force is needed for operating the adjustment device. The term "servo means" encompasses the normal definition of a servo mechanism, i.e. an automatic device that controls large amounts of power by means of very small amounts of power, but may alternatively or additionally encompass the definition of a mechanism that transfers a weak force acting on a moving element having a long stroke into a strong force acting on another moving element having a short stroke. The servo means may comprise a motor, preferably an electric motor, which may be reversible.

According to the invention, a reverse servo is employed. The term "reverse servo" is to be understood as a mechanism that transfers a strong force acting on a moving element having a short stroke into a weak force acting on another moving element having a long stroke; i.e. the opposite function of the above-defined alternative mechanism of a normal servo mechanism. A first closed hydraulic system that controls another closed hydraulic system in which hydraulic means of the adjustment device is incorporated may be used. Minor changes in the amount of fluid in a smaller reservoir of the first system could then be transferred by the reverse servo into major changes in the amount of fluid in a larger reservoir in the second system. In consequence, the change of volume in the larger reservoir of the second system will affect the hydraulic means of the adjustment device. For example, a short stroke that decreases the volume of the smaller reservoir will cause the larger reservoir to supply the adjustment device with a large amount of hydraulic fluid, which in turn results in a long mechanical adjustment stroke on the restriction device. The great advantage of using such a reverse servo is that the larger volume system could be placed inside the abdomen or retroperitoneum where there is more space and still it would be possible to use manual manipulation means of the smaller system subcutaneously. The smaller reservoir could be controlled directly or indirectly by a fluid supply means. The fluid supply means includes another small reservoir, which may be placed subcutaneously and may be activated by manual manipulation means.

The reverse servo comprises hydraulic means and a main fluid supply reservoir and eventually an additional fluid supply reservoir. Both reservoirs define a chamber containing hydraulic fluid, and the hydraulic means comprises first and second wall portions of the main fluid supply reservoir, which are displaceable relative to each other to change the volume of the chamber of the main fluid supply reservoir. The hydraulic means may control the adjustment device indirectly, e.g. via an increased amount of fluid in the main fluid supply reservoir, in response to a predetermined first displacement of the first wall portion of any of the reservoirs relative to the second wall portion of the reservoir to restrict the blood flow leaving the penis, and to control the adjustment device in response to a second displacement of the first wall portion of any reservoir relative to the second wall portion, to indirectly adjust the restriction device to release the penile tissue. The wall portions of the reservoirs may be designed to be displaceable relative to each other by manual manipulation thereof or be displaceable relative to each other by manually pushing, pulling or rotating any of the wall portions of the reservoir in one direction. Alternatively, the wall portions of the main fluid supply reservoir may be displaceable relative to each other by magnetic means, hydraulic means or electric control means including an electric motor.

The magnetic means, hydraulic means, or electrical control means may all be activated by manually manipulated means preferably located subcutaneously. This control may be indirect for example via a switch.

Even in the broadest embodiment of the invention the adjustment device may comprise a servo means. The servo means may comprise a hydraulic operation means, an electrical control means, a magnetic means, mechanical means or a manual manipulation means. The hydraulic operation means, electrical control means, mechanical means or magnetic means may be activated by manual manipulating means. Using a servo system will save the use of force when adjusting the adjustment device which may be of importance in many applications, for example when a battery cannot put out enough current although the total energy in the battery is more than enough to power the system.

In accordance with a preferred embodiment of the invention, the apparatus comprises implantable electrical components including at least one, or only one single voltage level guard and a capacitor or accumulator, wherein the charge and discharge of the capacitor or accumulator is controlled by use of the voltage level guard. As a result, there is no need for any implanted current detector and/or charge level detector for the control of the capacitor, which makes the apparatus simple and reliable.

All solutions may be controlled by a wireless remote control for controlling the adjustment device. The remote control may advantageously be capable of obtaining information related to the blood flow leaving the penis or the blood pressure or other important physical parameters and of commanding the adjustment device to adjust the restriction device in response to obtained information. With the wireless remote control the apparatus of the invention is conveniently controlled by the patient when he so desires, which is of great advantage compared to the prior art procedures. With the remote control the apparatus of the invention is conveniently controlled to adjust the implanted restriction device, which controls the blood flow leaving the penis. The restriction device may be operable to open and close the blood flow passageway formed by the penile exit veins. The restriction device may steplessly control the cross-sectional area of the passageway.

The apparatus may further comprise a pressure sensor for directly or indirectly sensing the pressure against the restriction device and the restriction device may control the blood flow in response to signals from the pressure sensor. The pressure sensor may be any suitable known or conventional pressure sensor such as shown in U.S. patents 5540731, 4846181, 4738267, 4571749, 4407296 or 3939823; or an NPC-102 Medical Angioplasty Sensor. The adjustment device preferaby non-invasively adjusts the restriction device to change the size of the cross-sectional area.

The adjustment device and/or other energy consuming components of the apparatus may be energised with wirelessly transmitted energy from outside the patient's body or be powered by an implanted battery or accumulator.

The apparatus may further comprise an implanted energy transforming device for transforming wireless energy directly or indirectly into kinetic energy for operation of the restriction device. The energy transforming device may, preferably directly, transform the wireless energy in the form of sound waves into electric energy for operation of the restriction device. Suitably the energy transforming device comprises a capacitor adapted to produce electric pulses from the transformed electric energy.

The apparatus of the invention may further comprise an energy transfer means for wireless transfer of energy from outside the patient's body to the adjustment device and/or other energy consuming implantable components of the apparatus. The energy transfer means may be adapted to intermittently transfer the energy, preferably electric energy, in the form of a train of energy pulses for direct use in connection with the energising of the energy consuming components of the apparatus. An implanted capacitor having a capacity less than 0,1 µF may be used for producing the train of energy pulses.

A motor may be implanted for operating the adjustment device, wherein the energy transfer means is adapted to directly power the motor with transferred energy. Alternatively, or in combination with the motor, a pump may be implanted for operating the adjustment device, wherein the energy transfer means is adapted to transfer wireless energy in the form of electromagnetic waves for direct power of the pump. Preferably, the pump is not a plunger type of pump, but may comprise a peristaltic or membrane pump.

The energy transfer means preferably transfers wireless energy in the form of electromagnetic waves. However, for safety radio waves may be excluded.

Alternatively, the energy transferred by the energy transfer means may comprise an electric field or a magnetic field.

Most preferred, the energy transferred by the energy transfer means comprises a signal.

Preferably, the wireless remote control comprises a separate signal transmitter or receiver and a signal receiver or transmitter implanted in the patient. For example, the signal transmitter and signal receiver may transmit and receive a signal in the form of digital pulses, which may comprise a magnetic or electric field. Alternatively, which is preferred, the signal transmitter and signal receiver may transmit and receive an electromagnetic wave signal, a sound wave signal or a carrier wave signal for a remote control signal. The receiver may comprise an implanted control unit for controlling the adjustment device in response to a control signal from the signal transmitter.

The apparatus of the invention may further comprise an implanted energiser unit for providing energy to energy consuming implanted components of the apparatus, such as electronic circuits and/or a motor for operating the adjustment device. The apparatus may comprise an external energy transmitter for transmitting wireless energy, wherein the energiser unit is adapted to transform the wireless energy into electric energy. An implanted electric motor may operate the adjustment device and the energiser unit may be adapted to power the electric motor with the electric energy transformed from the wireless energy.

The energiser unit may comprise a battery and a switch operable by the wireless energy transmitted by the external transmitter, for connecting the battery to the implanted energy consuming components of the apparatus in an "on" mode when the switch is powered by the wireless energy and to keep the battery disconnected from the energy consuming components in a "standby" mode when the switch is not powered.

The control unit may power such an implanted motor with energy provided by the energiser unit in response to a control signal received from the signal transmitter. Any known or conventional signal transmitter or signal receiver that is suitable for use with a human or mammal patient may be provided as the signal transmitter or signal receiver of the invention.

Generally, all the signals mentioned above may comprise electromagnetic waves, such as infrared light, visible light, laser light, micro waves, or sound waves, such as ultrasonic waves or infrasonic waves, or any other type of wave signals. The signals may also comprise electric or magnetic fields, or pulses. All of the above-mentioned signals may comprise digital signals. The control signals may be carried by a carrier wave signal, which in an alternative embodiment may be the same signal as the wireless energy signal. Preferably a digital control signal may be carried by an electromagnetic wave signal. The carrier wave or control signal may be amplitude or frequency modulated.

The motor may be any type of motor, such as a pneumatic, hydraulic or electric motor and the energiser unit may power the motor with pressurized gas or liquid, or electric energy, depending on the type of motor. Where the motor is an electric motor, it may power pneumatic or hydraulic equipment.

The energiser unit may comprise a power supply and the control unit may power the motor with energy from the power supply. Preferably, the power supply is an electric power supply, such as a battery, and the motor is an electric motor. In this case, the battery also continuously powers at least part of the circuitry of the signal receiver in a standby mode between the adjustments, in order to keep the signal receiver prepared for receiving signals transmitted from the signal transmitter.

The energiser unit may transfer energy from the control signal, as the control signal is transmitted to the signal receiver, into electric energy for powering the implanted electronic components. For example, the energiser unit may transfer the energy from the control signal into a direct or alternating current.

In case there is an implanted electric motor for operating the adjustment device the energiser unit may also power the motor with the transferred energy. Advantageously, the control unit directly powers the electric motor with electric energy, as the energiser unit transfers the signal energy into the electric energy. This embodiment is particularly simple and does not require any recurrent invasive measures for exchanging empty power supplies, such as batteries, that is required in the first embodiment described above. The motor may also be directly powered with wirelessly transmitted electromagnetic or magnetic energy in the form of signals, as the energy is transmitted. All the various functions of the motor and associated components described in the present specification may be used where applicable.

For adjustment devices of the type that requires more, but still relatively low, power for its operation, the energiser unit may comprise a rechargeable electric power supply for storing the electric energy obtained and the control unit may power the electric motor with energy from the rechargeable electric power supply in response to a control signal received from the signal transmitter. In this case, the rechargeable power supply can be charged over a relatively long time (e.g. a few seconds up to a half hour) without powering the electric motor.

The electric power supply suitably comprises an inexpensive simple capacitor. In this case, the electric motor may be a stepping motor. In all embodiments the motor may preferable be able to perform a reversing function.

The signal transmitter may transmit an electromagnetic signal and the energiser unit may draw radiant energy from the electromagnetic wave signal, as the latter is transmitted to the signal receiver, and transfer the radiant energy into electric energy.

Alternatively, the energiser unit may comprise a battery or accumulator, an electrically operable switch adapted to connect the battery to the signal receiver in an on mode when the switch is powered and to keep the battery disconnected from the signal receiver in a standby mode when the switch is not powered, and a rechargeable electric power supply for powering the switch. The control unit may power the electric motor with energy from the battery in response to a control signal received from the signal transmitter, when the switch is in its on mode. Advantageously, the energiser unit may transform wave energy from the control signal, as the latter is transmitted to the signal receiver, into a current for charging the rechargeable electric power supply, which suitably is a capacitor. Energy from the power supply is then used to change the switch from off (standby mode) to on. This embodiment is suited for adjustment devices of the type that require relatively high power for their operation and has the advantage that the electronic circuitry of the signal receiver does not have to be powered by the battery between adjustments. As a result, the lifetime of the battery can be significantly prolonged. The switch may be switched manually or by the use of magnetic or electric energy.

As an example, the signal transmitter may transmit an electromagnetic wave signal and the energiser unit may draw radiant energy from the electromagnetic wave signal, as the latter is transmitted to the signal receiver, and may transfer the radiant energy into said current. The energiser unit suitably comprises a coil of the signal receiver for inducing an alternating current as the electromagnetic wave signal is transmitted through the coil and a rectifier for rectifying the alternating current. The rectified current is used for charging the rechargeable power source.

Alternatively, the signal transmitter and receiver may solely be used for a control signal and a further pair of signal transmitter and receiver may be provided for transferring signal energy to implanted components. By such a double system of signal transmitters and receivers the advantage is obtained that the two systems can be designed optimally for their respective purposes, namely to transmit a control signal and to transfer energy from an energy signal. Accordingly, the apparatus may further comprise an external energy transmitter for transmitting wireless energy, wherein the energiser unit comprises a battery and an operable switch for connecting the battery to the signal receiver in an "on" mode when the switch is powered and for keeping the battery disconnected from the signal receiver in a "standby" mode when the switch is not powered, and the external energy transmitter powers the switch. Suitably, the energy transmitter may directly power the switch with the wireless energy to switch into the "on" mode. As should be realized by a skilled person, in many of the above-described embodiments of the invention the adjustment device may be operated by control means or manual manipulation means implanted under the skin of the patient, such as a pump, an electrical switch or a mechanical movement transferring means. In the manual embodiment it is not necessary to use a motor for operating the adjustment device.

An injection port connected to the hydraulic means may be provided for enabling, normally single, once-and-for-all, calibration of the amount of fluid in the hydraulic system.

In all embodiments a motor may be operatively connected to the adjustment device. A reversing device may be implanted in the patient for reversing the motor. The adjustment device preferably adjusts the restriction device in a non-manual manner without the patient touching his skin.

The adjustment device may be adapted to hydraulically adjust the restriction device by using hydraulic means which is devoid of hydraulic fluid of the kind having a viscosity that substantially increases when exposed to heat or a magnetic field, i.e. the hydraulic fluid would not become more viscous when exposed to heat or influenced by magnetic forces.

All the above-described various components, such as the motor, pump and capacitor, may be combined in the different embodiments where applicable. Also the various functions described in connection with the above embodiments of the invention may be used in different applications, where applicable.

All the various ways of transferring energy and controlling the energy presented in the present specification may be practised by using all of the various components and solutions described.

The invention may be used in a method for treating male sexual impotence, comprising surgically implanting in the body of a male patient suffering from sexual impotence an adjustable restriction device which directly engages a portion of the normal penile tissue of the patient to affect the blood flow leaving the penis and the prolongation thereof, and when desired to achieve erection, mechanically adjusting the restriction device to temporarily restrict the blood flow leaving the penis.

The method may further comprise implanting the male sexual impotence treatment apparatus in the base of the patient's penis or its prolongation, preferably implanting an restriction device engaging the corpora cavernosa, crura or the prolongation thereof as a single unit or engaging the two corpora cavernosa or crura or the prolongations thereof separately. The method may comprise implanting the restriction device engaging one or both of the crura of the penis.

As a modification, the method may further comprise implanting the restriction device engaging at least one of the exit veins from the penis

The invention may also be used in another method for treating male sexual impotence, comprising the steps of: placing at least two laparascopical trocars in the body of a male patient suffering from sexual impotenc, inserting a dissecting tool through the trocars and dissecting an area of the penis and abdominal or peritoneal surroundings, placing at least one adjustable restriction device in the dissected area engaging the penile tissue or the prolongation thereof, and adjusting the restriction device to restrict the blood flow leaving the penis when the patient wishes to achieve erection.

The method may further comprise mechanically adjusting said restriction device in a non-manual manner. The restriction device may engage (a) both of the corpora cavernosa, the crura of the penis or the prolongation thereof as a single unit; or (b) one of the exit veins from the penis.

Alternatively, the method may further comprise implanting (a) a further adjustable restriction device, wherein the two restriction devices engage the two corpora cavernosa, the crura of the penis or their prolongations, respectively, as separate units; or (b) several restriction devices engaging respective exit veins from the penis.

The method may further comprise implanting a source of energy in the patient and providing a control device for controlling the source of energy from outside the patient's body to supply energy to the restriction device.

The invention is described in more detail in the following with reference to the accompanying drawings, in which
FIGURE 1 is a schematic sectional view of a male sexual impotence treatment apparatus;
FIGURES 2 and 3 are cross-sectional views taken along the lines II-II and III-III, respectively, of FIGURE 1;
FIGURES 4 and 5 schematically show two alternative designs of the embodiment of FIGURE 1;
FIGURE 6 schematically illustrates a motor arrangement for the design according to FIGURE 5;
FIGURE 7 is a schematic sectional view of a second apparatus;
FIGURE 8 schematically illustrates a hydraulic transmision conduit for the apparatus of FIGURE 7;
FIGURE 9 is a schematic sectional view of a third apparatus
FIGURE 10 is a modification of the apparatus of FIGURE 9;
FIGURE 11 is a schematic view of a fourth apparatus;
FIGURES 12 and 13 are enlarged details of the apparatus of FIGURE 11;
FIGURE 14 is a cross-section along the line XIV-XIV of FIGURE 11;
FIGURE 15 is a schematic view of a fifth apparatus;
FIGURE 16 is an enlarged detail of FIGURE 15;
FIGURE 17 is a cross-section along the line XVII-XVII of FIGURE 15;
FIGURES 18 to 21 are schematic sectional views of a sixth, seventh, eighth and ninth, apparatus;
FIGURES 22 and 23 illustrate a fully open and a reduced restriction opening, respectively, of the apparatus of FIGURE 21;
FIGURE 24 is a schematic view of a tenth apparatus;
FIGURE 25 is an enlarged detail of the apparatus of FIGURE 24;
FIGURES 26 and 27 illustrate a fully open and a reduced restriction opening, respectively, of the apparatus of FIGURE 24;
FIGURE 28 schematically illustrates a cushion arrangement for protecting the tissue of the patient;
FIGURE 29A-D is a block diagram of four different principal hydraulic arrangements;
FIGURE 30A-D are cross-sectional views of a pump mechanism according to FIGURE 29C, which pumps fluid in opposite directions by mechanically pushing a wall portion in only one direction;
FIGURE 31 is a cross-sectional view of a reservoir having a variable volume controlled by a remote control motor, in accordance with the principal arrangement shown in FIGURE 29B or 30B; the principal
FIGURE 32 is a cross-sectional view of a reservoir having a variable volume adjustable by manual manipulation, in accordance, with the principal arrangement shown in FIGURE 29B or 29D;
FIGURE 33A is a front view of a hydraulic, pneumatic or mechanical reverse servo system in accordance with the principal embodiment shown in FIGURE 29D;
FIGURE 33B is a cross-sectional view taken along line VB-VB of FIGURE 33A;
FIGURE 34 is a block diagram illustrating remote control components of the apparatus of the invention;
FIGURE 35 is a schematic view of a circuitry used for the system of the block diagram of FIGURE 34;
FIGURES 36A and 36B are schematic views of an eleventh apparatus;
FIGURES 37A and 37B are schematic views of a twelfth apparatus;
FIGURE 38 is a schematic view of a thirteenth apparatus;
FIGURES 39A, 39B and 39C are a schematic front view and schematic sectional views, respectively, of a fourteenth apparatus;
FIGURES 40A through 44B are five modifications of the apparatus of FIGURES 39A-39C;
FIGURE 45 illustrates the apparatus of the invention with a restriction device implanted around the corpus cavernosum of a patient; and
FIGURE 46 illustrates the apparatus of the invention with two restriction members implanted around respective exit veins from the penis of a patient.

Referring to the drawing figures, like reference numerals designate identical or corresponding elements throughout the several figures.

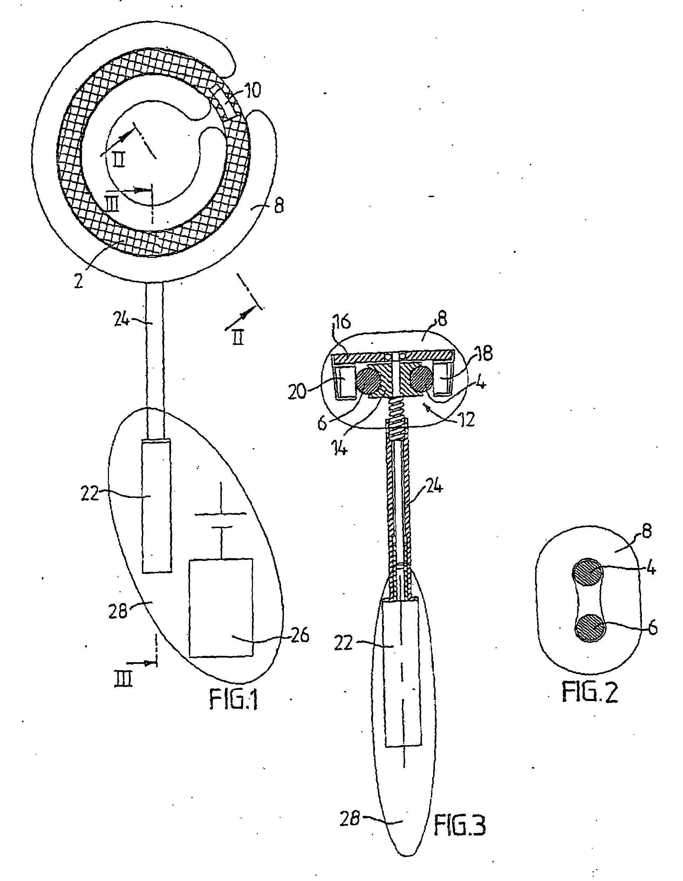

FIGURES 1-3 show an example of a male sexual impotence treatment apparatus comprising a restriction device having an elongated restriction member in the form of a circular resilient core 2 with two overlapping end portions 4,6. The core 2 defines a substantially circular restriction opening and is enclosed in an elastic soft hose 8 except at a releasable and lockable joint 10 of the core 2, which when released enables application of the core 2 with its hose 8 around a tissue of a patient, such as the corpus cavernosum or one or more exit veins from the patient' s penis. The materials of all of these elements are bio-compatible so that the patient's body will not reject them. A mechanical adjustment device 12 for mechanically adjusting the longitudinal extension of the core 2 to change the size of the restriction opening comprises a drive wheel 14 in frictional engagement with the overlapping end portions 4,6 of the core 2. The drive wheel 14 is journalled on a holder 16 placed in the hose 8 and provided with two counter pressure rollers 18,20 pressing the respective end portions 4, 6 of the core 2 against the drive wheel 14 to increase the frictional engagement therebetween. An electric motor 22 is connected to the drive wheel 14 via a long flexible drive shaft 24 and is moulded together with a remote controlled power supply unit 26 in a body 28 of silicone rubber. The length of the flexible drive shaft 34 is selected so that the body 28 can be placed in a desired position in the patient's body, suitably in the abdomen.

When the patient wishes to achieve erection, he controls the power supply unit 26 to power the electric motor 22 to turn the drive wheel 14 in one direction to reduce the diameter of the core 2, so that the penile tissue is squeezed and the blood flow leaving the penis is restricted. When the patient wishes to regain flaccid condition of his penis, he controls the power supply unit 26 to power the electric motor 22 to turn the drive wheel 14 in the opposite direction to increase the diameter of the core 2, so that the tissue is released.

Alternatively, a rack gear may be formed on one of the end portions 4,6 of the core 2 and the drive wheel 14 may be replaced by a drive gear wheel connected to the other end portion of the core 2 and in mesh with the rack gear.

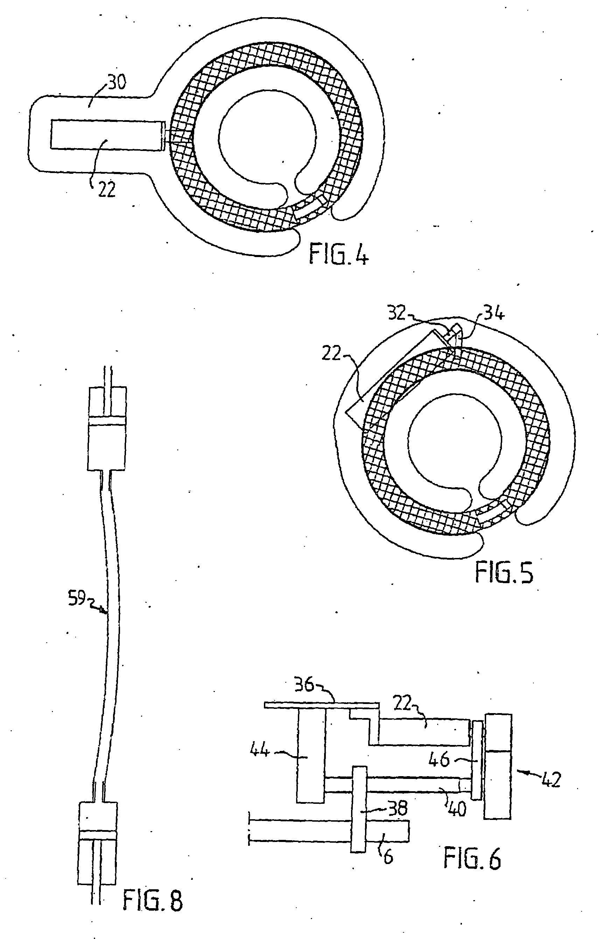

FIGURE4 shows an example example which is identical to the example of FIGURES 1-3, except that the motor 22 is encapsulated in a lateral protrusion 30 of the hose 8 so that it is fixed to the core 2 and has a short drive shaft 32 onto which the drive wheel 14 is mounted, and that the motor 22 is positioned relative to the circular core 2 such that the drive shaft 32 extends radially thereto.

FIGURE 5 shows an example which likewise is identical to the example of FIGURES 1-3, except that the motor 22 is encapsulated in the hose 8 so that it is fixed to the core 2 and has a short drive shaft 32, and that the motor 22 is positioned relative to the core 2 such that the drive shaft 32 extends substantially tangentially to the circular core 2. There is an angular gearing 34 connecting the drive shaft 32 to the drive wheel 14.

FIGURE 6 shows a suitable arrangement for the motor 22 in the example of FIGURE 5, comprising a first clamping member 36 secured to one end portion of the core 2 and a second clamping member 38 secured to the other end portion 6 of the core 2. The motor 22 is secured to the first clamping member 36 and is operatively connected to a worm 40 via a gear transmission 42. The worm 40 is journalled at its opposite ends on holders 44 and 46, which are rigidly secured to the clamping member 36 and the motor 22, respectively. The second clamping member 38 has a pinion in mesh with the worm 40. When the motor 22 is powered the worm 40 rotates and will thereby pull the end portion 6 of the core 2 in one or the opposite longitudinal direction, so that the diameter of the substantially circular core 2 is either increased or decreased.

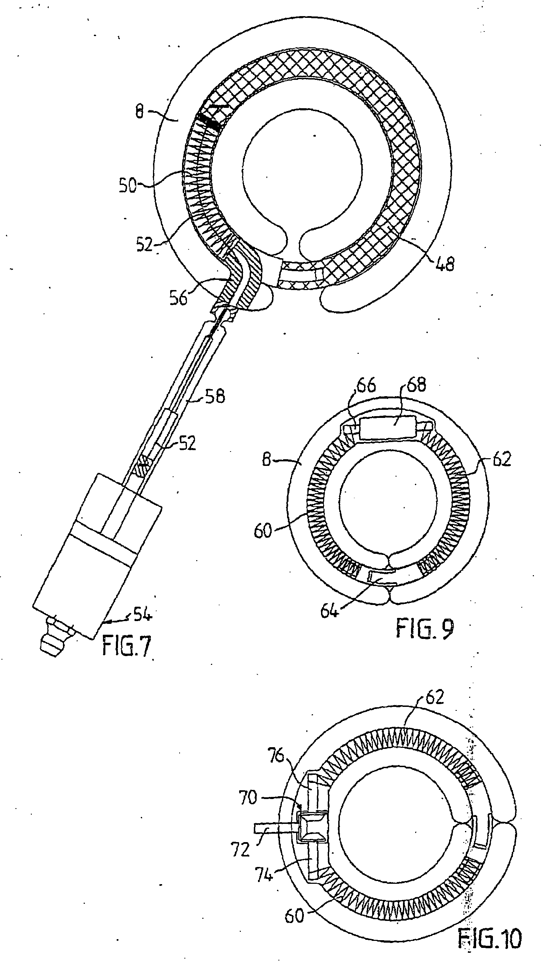

FIGURE 7 shows an example in which the elongated restriction member comprises a core 48 and a helical spring 50. A spring contracting means in the form of a flexible pulling member 52, i.e. a string, wire or cable, is connected to the core 48 at one end thereof and extends through the helical spring 50. A hydralic motor in the form of a cylinder/piston unit 54 is adapted to pull the flexible pulling member 52 to contract the helical spring 50 against an arresting member 56, which is fixed relative to the core 48. A tube 58 hinged to the arresting member 56 extends between the cylinder/piston unit 54 and the arresting member 56, the flexible pulling member 52 running through the tube 58 and being connected to the piston of the cylinder/piston unit 54. FIGURE 8 shows a similar example in which a hydraulic transmission conduit 59 is provided between two piston-cylinder assemblies 54, for use as the hydraulic motor/device in FIGURE 7.

FIGURE 9 shows an example in which the restriction member comprises two elongated helical springs 60 and 62 having free ends, and a body 64 to which the springs 60,62 are nonrotatably secured at their opposite ends. The body 64 comprises two separate parts secured to opposite end portions of the enclosing elastic hose 8 and is designed with a releasable and lockable joint between the separate parts. An adjustment device in the form of a drive shaft 66 has two opposite end portions connected to the helical springs 60,62, respectively, at their free ends. The coils of the springs 60,62 form left and right hand helices, respectively. A motor 68 is adapted to rotate the drive shaft 66 in one direction to enlarge the coils of the helical springs 60,62 to longitudinally contract the springs 60,62 and to rotate the drive shaft 66 in the opposite direction to reduce the size of the coils of the springs 60,62 to longitudinally extend the springs 60,62. Thus, the elongated helical springs 60,62 defines a restriction opening, the size of which is increased when the springs 60,62 are extended and decreased when the springs 60,62 are contracted.

FIGURE 10 shows an example which is identical to the example of FIGURE 9, except that the adjustment decice comprises a gearing having an input shaft 72 and two opposite aligned output shafts 74 and 76 connected to the helical springs 60 and 62, respectively, at their free ends. The input shaft 72 is connected to the output shafts 74,76 such that they rotate at opposite directions upon rotation of the input shaft 72. The coils of the springs 60, 62 form the same helices.

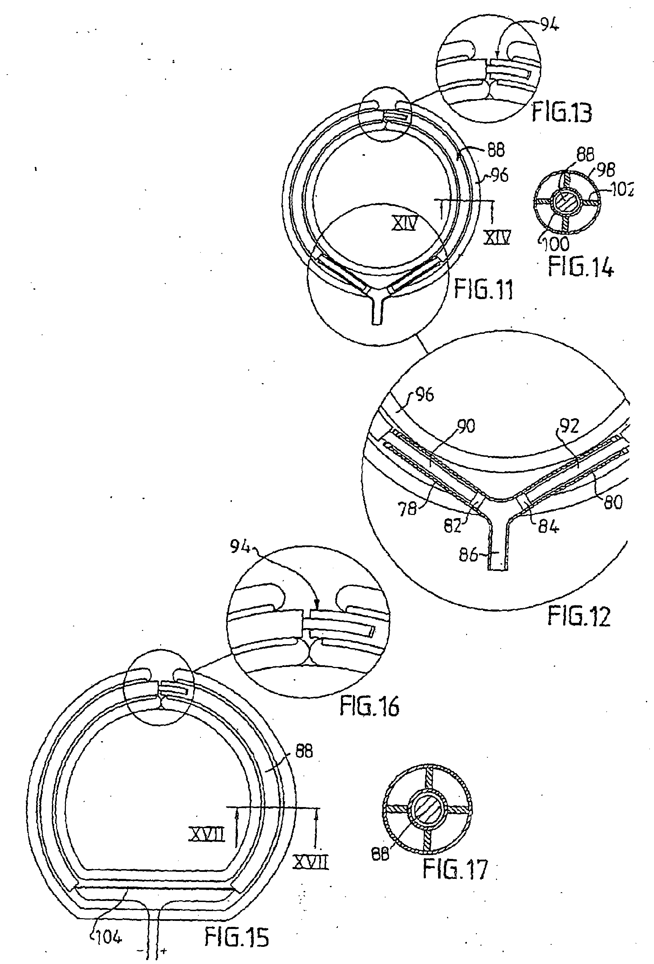

FIGURES 11-14 show an example in which a hydraulic motor comprises two interconnected cylinders 78 and 80 and two pistons 82 and 84 in the respective cylinders 78,80. The cylinders 78,80 have a common fluid supply inlet member 86, which together with the cylinders 78,80 takes the shape of a Y-pipe. The restriction member comprises an elongated resilient arcuate core 88. The adjustment device comprises two bars 90 and 92 secured to opposite ends of the core 88 and connected to the pistons 82 and 84, respectively. The core 88 defines a restriction opening and is provided with a releasable and lockable joint 94 (FIGURE 13) to permit application of the core 88 around the tissue. The core 88 and the cylinders 90,92 are enclosed by a soft elastic hose 96 except at the joint 94 and the inlet member 86. The hose 96 has an outer tubular wall 98 and a central coaxial inner tubular wall 100, which is fixed to the outer wall 98 by spoke members 102 (FIGURE 14). The core 88 is loosely fit in the inner tubular wall 100. By supplying fluid to or withdrawing fluid from the inlet 86 the pistons 82 and 84 will move towards or from each other, so that the restriction opening defined by the core 88 is changed by the longitudinal displacement of the bars 90,92.

FIGURES 15-17 show an example which is identical to the example of FIGURES 11-14, except that the adjustment device comprises an elongated voltage responsive element 104 secured to the opposite ends of the core 88, so that the core 88 and the element 104 form the restriction member. The element 104 is capable of bending inwardly into a bow in response to a voltage applied across the element 104. The radius of curvature of said bow is adjustable by changing the level of the voltage applied to element 104.

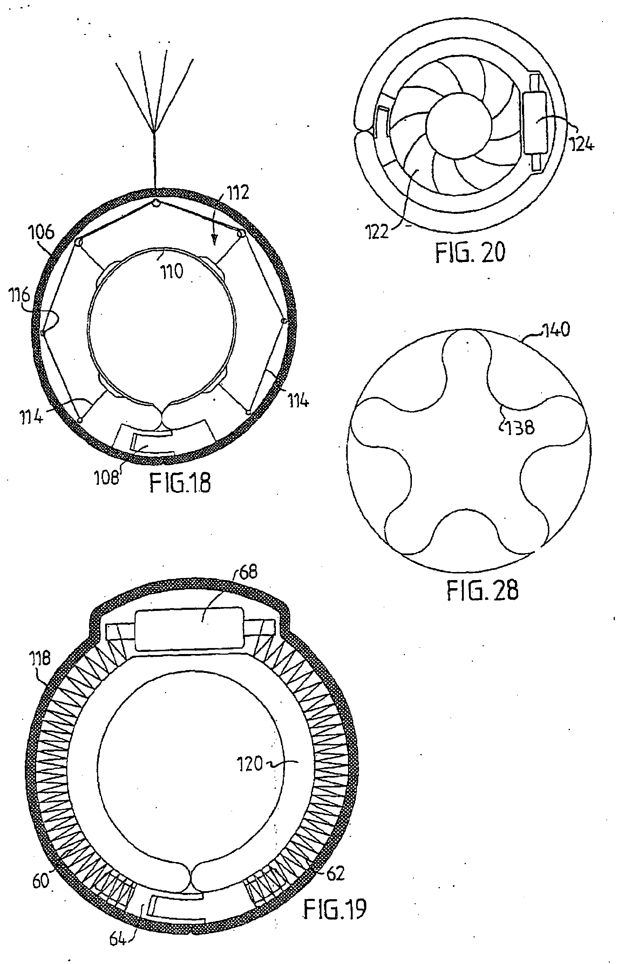

FIGURE 18 shows an example comprising a loop forming means in the form of a substantially rigid outer circular element 106 with a releasable and lockable joint 108. In this example the restriction member comprises an elastic inner circular element 110 formed by the innermost wall portion of an elastic hose 112 extending along the outer element 106. The inner circular element 110 is disposed concentrically within the outer circular element 106. The adjustment device comprises a plurality of threads 114 secured to the elastic inner element 110 along the circumference thereof and running from the inner element 110 via guide members 116 attached to the outer element 106. By pulling all the threads 114 the inner elastic element 110 is pulled under expansion radially outwardly towards the outer element 106. FIGURE 19 shows an example which is identical to the example of FIGURE, 9, except that it comprises a loop forming means in the form of a substantially rigid outer circular element 118 supporting the helical springs 60,62, and a soft elastic inner wall 120 extending along the springs 60,62. When the motor 68 rotates the helical springs 60, 62 in a direction that enlarges the coils of the springs 60,62, the coils are forced by the rigid outer element 118 to expand radially inwardly thereby reducing the size of the restriction opening formed by the circumferential confinement surface of the restriction member (springs 60,62 and body 64).

FIGURE 20 shows an example in which a restriction member comprises a plurality of arcuate lamellae 122 arranged like the conventional adjustable aperture mechanism of a camera. The adjustment device, not shown, is conventional and is operated by a motor 124 to adjust the lamellae 122 to change the size of an restriction opening defined by the lamellae 122.

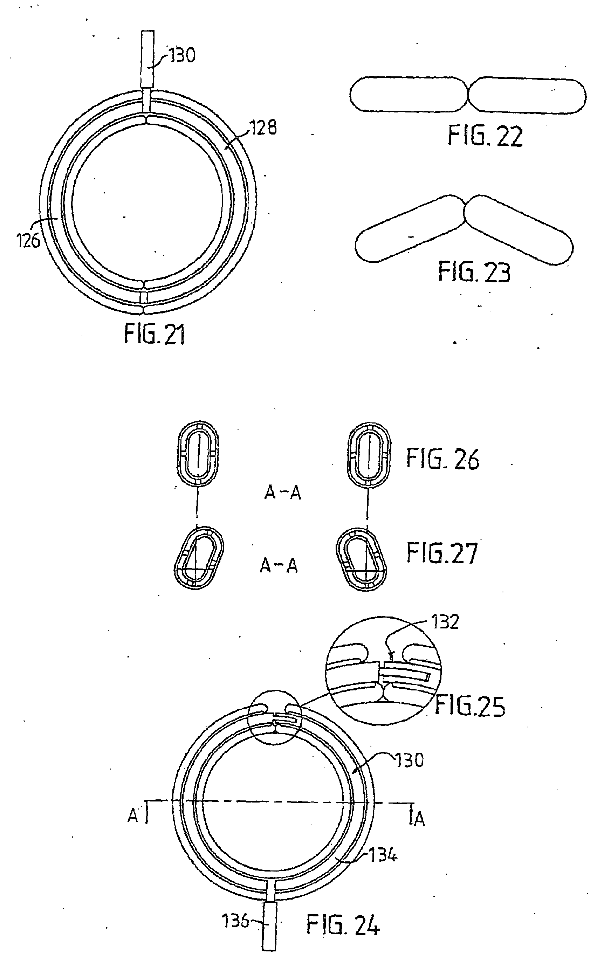

FIGURES 21-23 show an example in which a restriction member comprises two semi-circular elements 126 and 128 which are hinged together such that the semi-circular elements 126,128 are swingable relative to each other between a fully open state in which they substantially form a circle, illustrated in FIGURE, 22 and an angular state, in which the size of the restriction opening defined by the semi-circular elements 126,128 is reduced, illustrated in FIGURE, 23. The adjustment device, not shown, is conventional and is operated by a motor 130 to swing the semi-circular elements 126,128 relative to each other.

FIGURES 24-27 show an example in which a restriction member comprises an elastic belt 130 forming a circle and having a substantially oval cross-section. The restriction member 130 is provided with a releasable and lockable joint 132. An elastic double walled hose 134 encloses the belt 130 except at the joint 132. The adjustment device, not shown, is conventional and is operated by a motor 136 to turn the belt 130 around the longitudinal extension thereof between a fully open state, in which the inner broader side of the belt 130 forms a substantially cylindrical surface, illustrated in FIGURE 26, and a reduced open state, in which the inner broader side of the belt 130 forms a substantially conical surface, illustrated in FIGURE 27.

FIGURE 28 schematically illustrates a cushion arrangement for protecting the penile tissue, comprising a plurality of cushions 138 disposed in series along a substantially circular holding member 140. This cushion arrangement may be utilized in any of the above described embodiments of the invention.

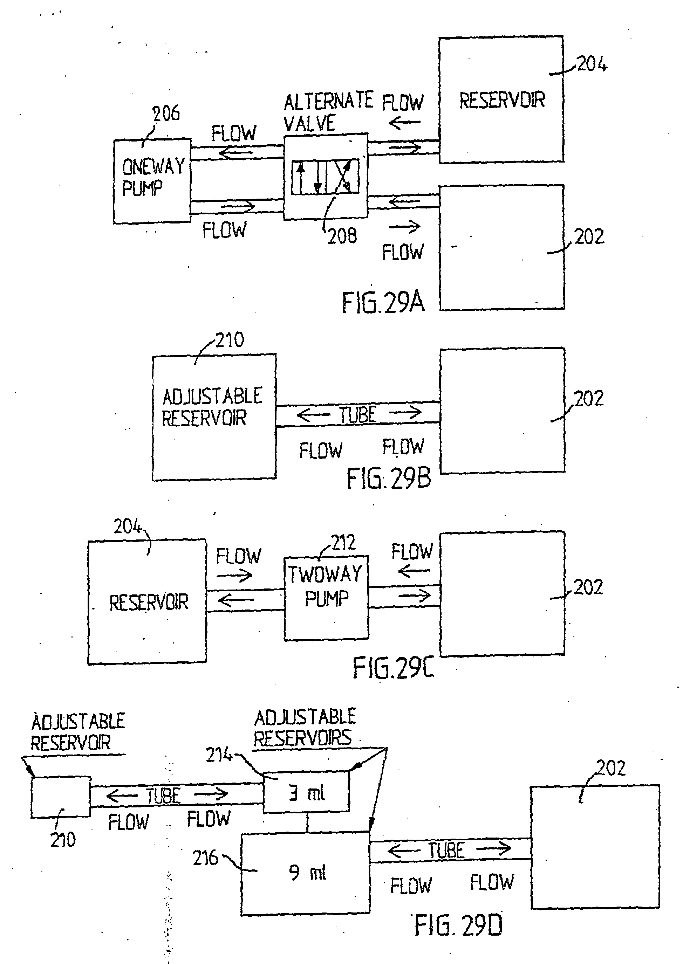

FIGURES 29A-D provide a block diagram of four different hydraulic transmission configurations. FIGURE 29A shows an adjustment device 202, a separate reservoir 204, a one way pump 206 and an alternate valve 208. FIGURE 29B shows the adjustment device 202 and an adjustable reservoir 210. FIGURE 29C shows the adjustment device 202, a two-way pump 212 and the reservoir 204. FIGURE 29D illustrates the present invention and shows a reverse servo system with a first closed system controlling a second system. The servo system comprises an adjustable servo reservoir 210 and a passive adjustable fluid supply reservoir 214. Any of the reservoirs can be the active reservoir, either the servo reservoir 210 or the fluid supply reservoir 214. The reservoir 214 controls a larger adjustable reservoir 216, which is used for the operation of the adjustment device 202 for changing the restriction opening of the restriction member.

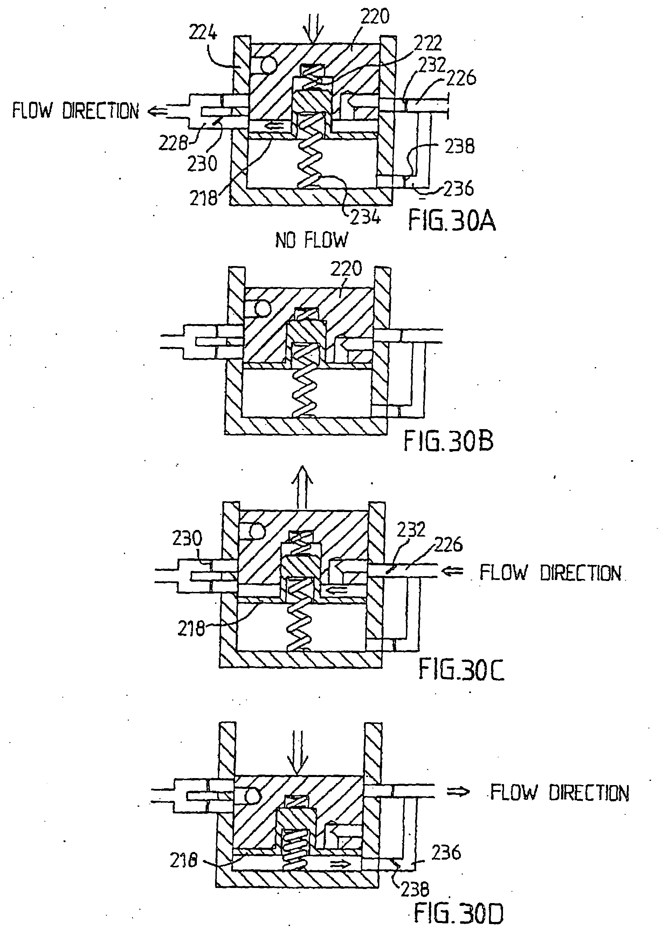

FIGURES 30A-D are cross-sectional views of a pump mechanism adapted to pump fluid in both directions only by mechanically pushing a separate sealing wall portion 218 in one direction. FIGURE 30A shows a piston 220 pushed forwards against a spring 222 towards the wall portion 218 and located in a pump housing 224 conducting fluid from a right upper fluid passage 226 of the housing 224 to a left fluid passage 228 of the housing 224. A main valve 230 is open and a nonreturn valve 232 is closed. FIGURE 30B illustrates the first pump movement in which the piston 220 has moved forwards and reaches the wall portion 218. FIGURE 30C illustrates how the piston 220 moves backwards by the action of the spring 222. The main valve 230 is now closed and the nonreturn valve 232 is open for fluid from the right upper passage 226. FIGURE 30D illustrates how the piston 220 is moved further downwards from its position according to FIGURE 30B while pushing the wall portion 218 downwards against a second spring 234 that is stronger than spring 222, so that fluid escapes from a right lower fluid passage 236. When moving the piston 220 backwards from the position of FIGURE 30D, fluid enters the left fluid passage 228 and a valve 238 in the lower right fluid passage 236 closes.

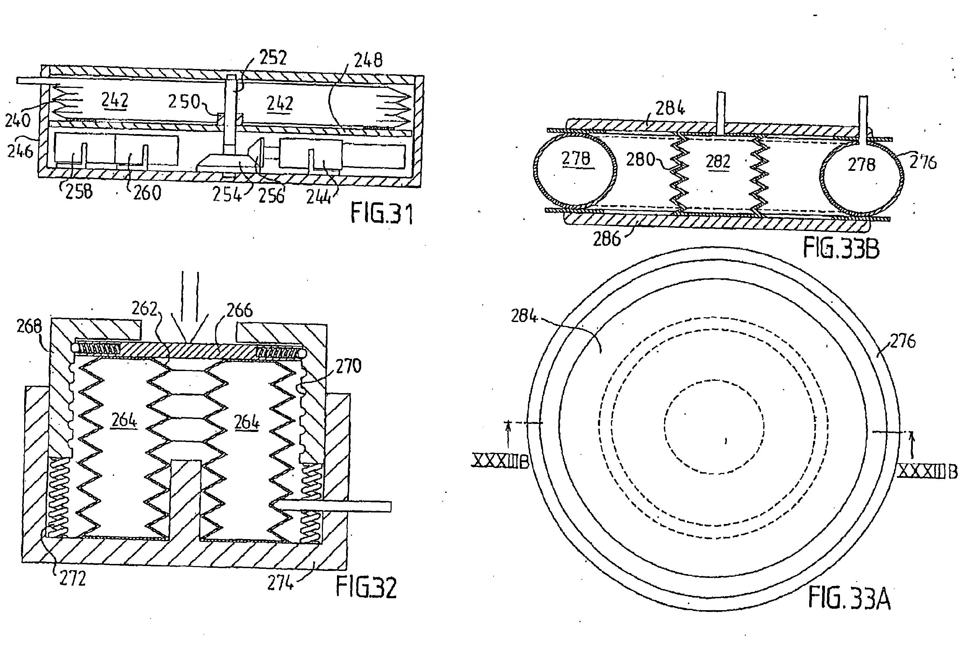

FIGURE 31 is a cross-sectional view of a reservoir 240 defining a chamber 242, the size of which is variable and is controlled by a remote controlled motor 244, in accordance with FIGURE 29B or 29D. The reservoir 240 and the motor 244 are placed in a housing 246. The chamber 242 is varied by moving a large wall 248. The wall 248 is secured to a nut 250, which is threaded on a rotatable spindle 252. The spindle 252 is rotated by the motor 244 via an angular gearing, which comprises two conical gear wheels 254 and 256 in mesh with each other. The motor 244 is powered by a battery 258 placed in the housing 246. A signal receiver 260 for controlling the motor 244 is also placed in the housing 246. Alternatively, the battery 258 and the signal receiver 260 may be mounted in a separate place. The signal receiver may comprise any known or conventional device which is capable of receiving a control signal and then operating the motor 244.

FIGURE 32 is a cross-sectional view of a reservoir 262 defining a chamber 264, the size of which is variable and is controlled by manual manipulation. A gable wall portion 266 of an open ended inner cylindrical housing 68 is adapted to be pushed downwards to fit in a desired locking groove 270 of a plurality of locking grooves 270 on the mantle wall of the cylindrical housing 268, to reduce the size of the chamber 64. The inner cylindrical housing 268 is suspended by springs 272 and is telescopically applied on an outer cylindrical housing 274. When pushing the inner cylindrical housing 268 it moves downwards relative to the outer cylindrical housing 274 causing the gable wall portion 266 to release from the locking groove 270 and move upwards relative to the inner cylindrical housing 268. When the inner housing 268 is moved upwardly by the action of the springs 272 the size of the chamber 264 is increased.

FIGURE 33A and 33B show a reverse servo means comprising a main ring-shaped fluid reservoir 276 defining a chamber 278, the size of which is variable. Centrally positioned in the main ring-shaped reservoir 276 there is a servo fluid reservoir 280 defining a chamber 282, the size of which is variable. The chamber 282 of the servo reservoir 280 is significantly smaller than the chamber 278 of the main reservoir 276. The two reservoirs 276 and 280 are situated between two opposite separate walls 284 and 286, and are secured thereto. When changing the amount of fluid in the servo reservoir 280, the two opposite walls 284,286 are moved towards or away from each other, whereby the size of the chamber 278 of the main reservoir 276 is changed.

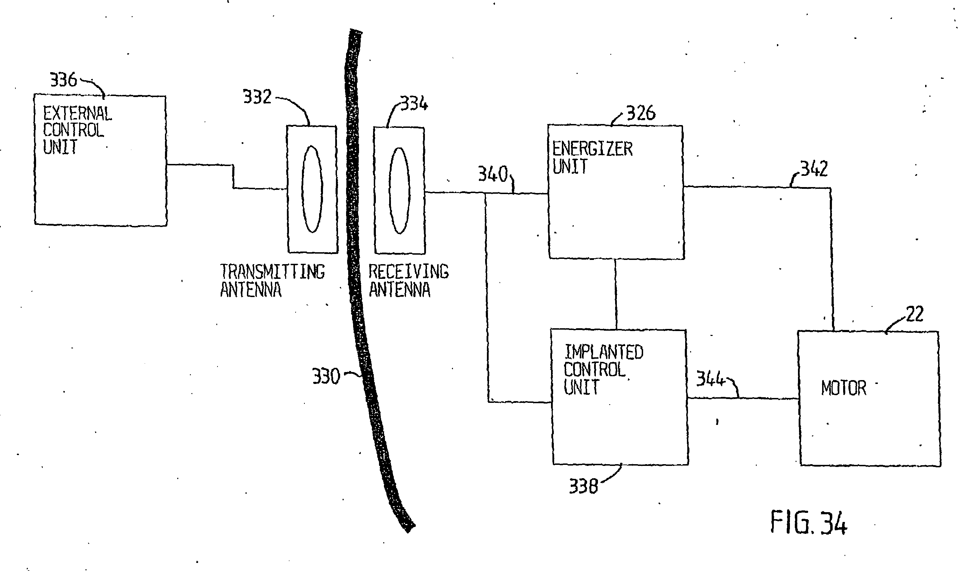

FIGURE 34 shows the basic parts of a remote control system of the apparatus of the invention including a motor, for instance the electric motor 22. In this case, the remote control system is based on the transmission of an electromagnetic wave signal, often of a high frequency in the order of 100 kHz - 1 gHz, through the skin 330 of the patient. In FIGURE 34, all parts placed to the left of the skin 330 are located outside the patient's body and all parts placed to the right of the skin 330 are implanted in the patient's body.

An external signal transmitting antenna 332 is to be positioned close to a signal receiving antenna 334 implanted in the patient's body close to the skin 330. As an alternative, the receiving antenna 334 may be placed for example inside the abdomen of the patient. The receiving antenna 334 comprises a coil, approximately 1-100 mm, preferably 25 mm in diameter, wound with a very thin wire and tuned with a capacitor to a specific high frequency. A small coil is chosen if it is to be implanted under the skin of the patient and a large coil is chosen if it is to be implanted in the abdomen of the patient. The transmitting antenna 332 comprises a coil having about the same size as the coil of the receiving antenna 334 but wound with a thick wire that can handle the larger currents that is necessary. The coil of the transmitting antenna 332 is tuned to the same specific high frequency as the coil of the receiving antenna 334.

An external control unit 336 comprises a microprocessor, a high frequency electromagnetic signal generator and a power amplifier. The microprocessor of the control unit 336 is adapted to switch on/off the generator and to modulate signals generated by the generator to send digital information via the power amplifier and the antennas 332,334 to an implanted control unit 338. To avoid that accidental random high frequency fields trigger control commands, digital signal codes are used. A keypad placed on the external control unit 336 is connected to the microprocessor thereof. The keypad is used to order the microprocessor to send a digital signal to either increase or decrease the size of the restriction opening defined by the loop of the restriction member (e.g. as described above). The microprocessor starts a command by applying a high frequency signal on the antenna 332. After a short time, when the signal has energised the implanted parts of the control system, commands are sent to increase or decrease the size of the restriction opening of the restriction member in predefined steps. The commands are sent as digital packets in the form illustrated below.

| | | | |
|---|---|---|---|
| Start pattern, 8 bits | Command, 8 bits | Count, 8 bits | Checksum, 8 bits |

The commands are sent continuously during a rather long time period (e.g. 30 seconds or more). When a new increase or decrease step is desired the Count byte is increased by one to allow the implanted control unit 338 to decode and understand that another step is demanded by the external control unit 336. If any part of the digital packet is erroneous, its content is simply ignored.

Through a line 340, an implanted energiser unit 326 draws energy from the high frequency electromagnetic wave signal received by the receiving antenna 334. The energiser unit 326 stores the energy in a power supply, such as a large capacitor, powers the control unit 338 and powers the electric motor 22 via a line 342.

The control unit 338 comprises a demodulator and a microprocessor. The demodulator demodulates digital signals sent from the external control unit 336. The microprocessor of the control unit 338 receives the digital packet, decodes it and, provided that the power supply of the energiser unit 326 has sufficient energy stored, sends a signal via a signal line 344 to the motor 22 to either increase or decrease the size of the restriction opening of the restriction member depending on the received command code.

Alternatively, the energy stored in the power supply of the energiser unit may only be used for powering a switch, and the energy for powering the motor 22 may be obtained from another implanted power source of relatively high capacity, for example a battery. In this case the switch is adapted to connect the battery to the control unit 338 in an "on" mode when said switch is powered by said power supply and to keep said battery disconnected from the control unit in a "standby" mode when the switch is not powered.

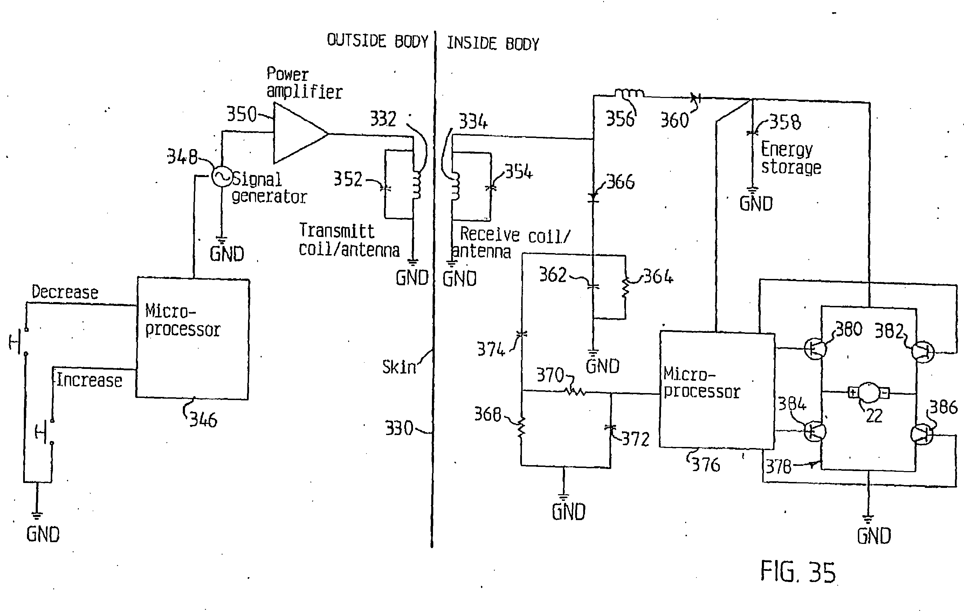

With reference to FIGURE 35, the remote control system schematically described above will now be described in accordance with a more detailed embodiment. The external control unit 336 comprises a microprocessor 346, a signal generator 348 and a power amplifier 350 connected thereto. The microprocessor 346 is adapted to switch the signal generator 348 on/off and to modulate signals generated by the signal generator 348 with digital commands that are sent to implanted components of the device of the invention. The power amplifier 350 amplifies the signals and sends them to the external signal transmitting antenna 332. The antenna 332 is connected in parallel with a capacitor 352 to form a resonant circuit tuned to the frequency generated by the signal generator 348.

The implanted signal receiving antenna coil 334 forms together with a capacitor 354 a resonant circuit that is tuned to the same frequency as the transmitting antenna 332. The signal receiving antenna coil 334 induces a current from the received high frequency electromagnetic waves and a rectifying diode 360 rectifies the induced current, which charges a storage capacitor 358. A coil 356 connected between the antenna coil 334 and the diode 360 prevents the capacitor 358 and the diode 360 from loading the circuit of the signal receiving antenna 334 at higher frequencies. Thus, the coil 356 makes it possible to charge the capacitor 358 and to transmit digital information using amplitude modulation.

A capacitor 362 and a resistor 364 connected in parallel and a diode 366 forms a detector used to detect amplitude modulated digital information. A filter circuit is formed by a resistor 368 connected in series with a resistor 370 connected in series with a capacitor 372 connected in series with the resistor 368 via ground, and a capacitor 374, one terminal of which is connected between the resistors 368,370 and the other terminal of which is connected between the diode 366 and the circuit formed by the capacitor 362 and resistor 364. The filter circuit is used to filter out undesired low and high frequencies. The detected and filtered signals are fed to an implanted microprocessor 376 that decodes the digital information and controls the motor 22 via an H-bridge 378 comprising transistors 380,382,384 and 386. The motor 22 can be driven in two opposite directions by the H-bridge 378.

The microprocessor 376 also monitors the amount of stored energy in the storage capacitor 358. Before sending signals to activate the motor 22, the microprocessor 376 checks whether the energy stored in the storage capacitor 358 is enough. If the stored energy is not enough to perform the requested operation, the microprocessor 376 waits for the received signals to charge the storage capacitor 358 before activating the motor 22.

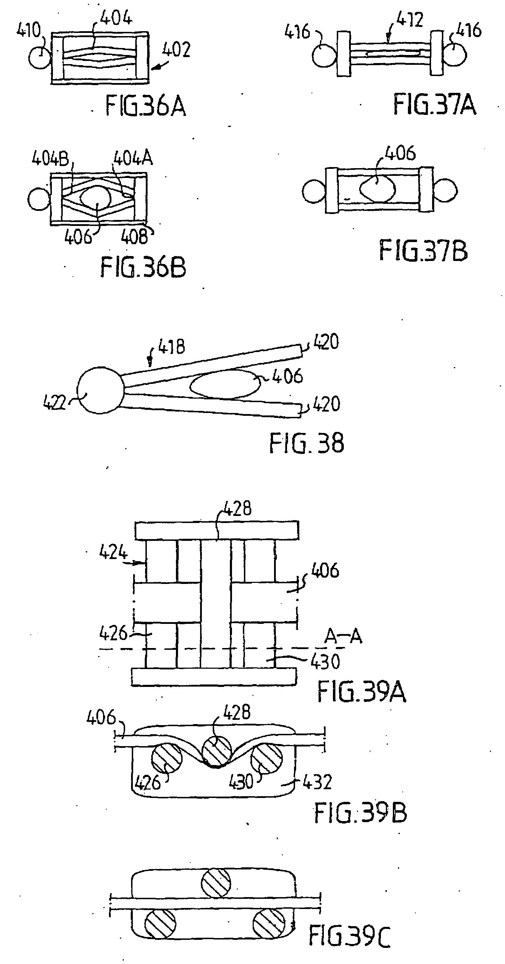

FIGURES 36A and 36B show an example comprising a restriction device 402 having an elongated flexible restriction member 404, such as a belt, a cord or the like. The flexible member 404 extends in a loop around the tissue, suitably an exit vein from the penis. (Alternatively, the flexible member 404 may comprise two separate parts on opposite sides of the vein.) One portion 404A of member 404 is attached to a frame 408 and another portion 404B of member 404 opposite portion 404A in the loop of the flexible member 404 is connected to an adjustment device 410, which is fixed to the frame 408. The adjustment device 410 pulls the flexible member 404 in the direction from portion 404A to squeeze the vein between two opposite lengths of the flexible member 404 to thereby restrict the blood flow in the vein 406, see FIGURE 36A, and releases the vein from the flexible member 404 to thereby increase the blood flow in the vein 406, see FIGURE 36B.

FIGURES 37A and 37B show an example comprising a restriction device 412 having two rigid plate or bar elements 414 on opposite sides of the vein 406. An adjustment device 416 moves the rigid elements 412 in parallel towards each other to squeeze the vein 406 between the rigid elements 412 to thereby restrict the blood flow in the vein 406, see FIGURE 37A, and moves the rigid elements 412 away from each other to release the vein 406, see FIGURE 37B.

FIGURE 38 shows an example comprising a restriction device 418 having two rigid articulated clamping elements 420 positioned on opposite sides of the vein 406. An adjustment device 422 turns the clamping elements 420 toward each other to clamp the vein 406 between the clamping elements 420 to thereby restrict the blood flow in the vein 406, and turns the clamping elements 420 away from each other to release the vein 406 from the clamping elements 420 to thereby increase the blood flow in the vein 406.

FIGURES 39A, 39B and 39C show an example comprising a restriction device 424 having three bending members in the form of cylindrical rollers 426, 428 and 430 displaced relative one another in a row along the vein 406 and positioned alternately on opposite sides of the vein 406. (Alternatively, each roller 426, 428 and 430 may take the shape of an hour-glass.) An adjustment device 432 moves the two outer rollers 426,430 laterally against the vein 406 in one direction and the intermediate roller 428 against the vein 406 in the opposite direction to bend the vein to thereby restrict the blood flow in the vein 406, see FIGURE 39B. To increase or restore the blood flow in the vein 406, the adjustment device 432 moves the rollers 426-430 away from the vein 406 to release the vein from the rollers 426-430, see FIGURE 39C.

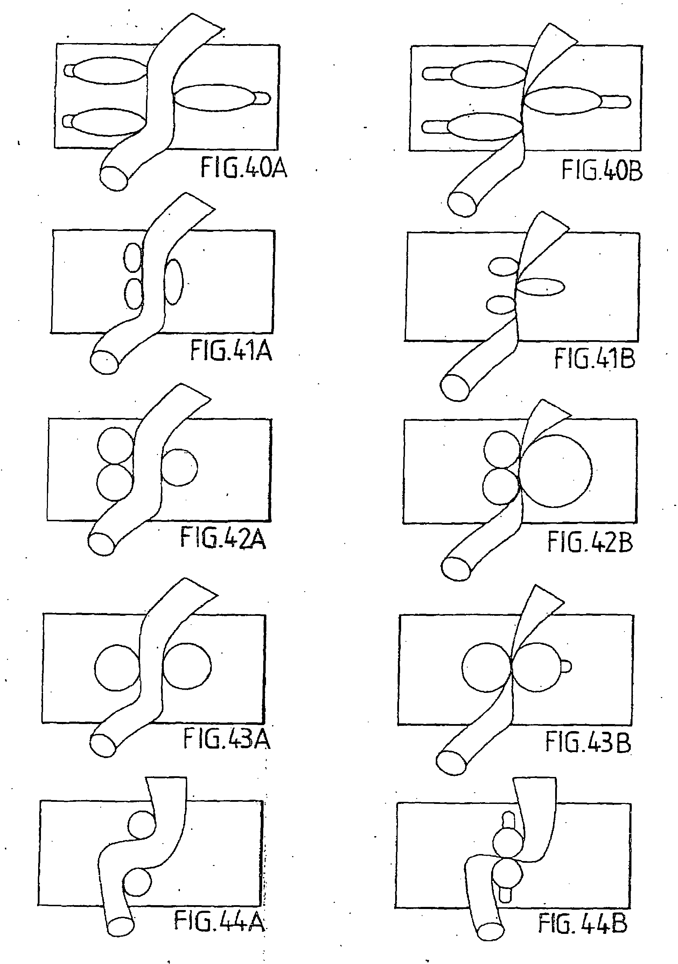

FIGURES 40A through 44B schematically illustrates modifications of the above example according to FIGURES 39A-39C. Thus, FIGURES 40A and 40B show an example similar to that of FIGURES 39A-39C except that the bending members are oval and not rotatable. FIGURES 41A and 41B show an similar to that of FIGURES 40A and 40B except that the oval bending members are rotatable to release the vein, see FIGURE 41A, and squeeze the vein, see FIGURE 41B. FIGURES 42A and 42B show an example similar to that of FIGURES 39A-39C except that the intermediate roller has a changeable diameter to release the vein, see FIGURE 42A, and squeeze .the vein, see FIGURE 42B. FIGURES 43A and 43B show an example similar to that of FIGURES 37A-37C except that the rigid elements are replaced by two cylindrical rollers positioned on opposite sides of the vein. Finally, FIGURES 44A and 44B show an example substantially similar to that of FIGURES 43A and 43B except that the restriction device is curved to form an S-shaped curvature of the vein.

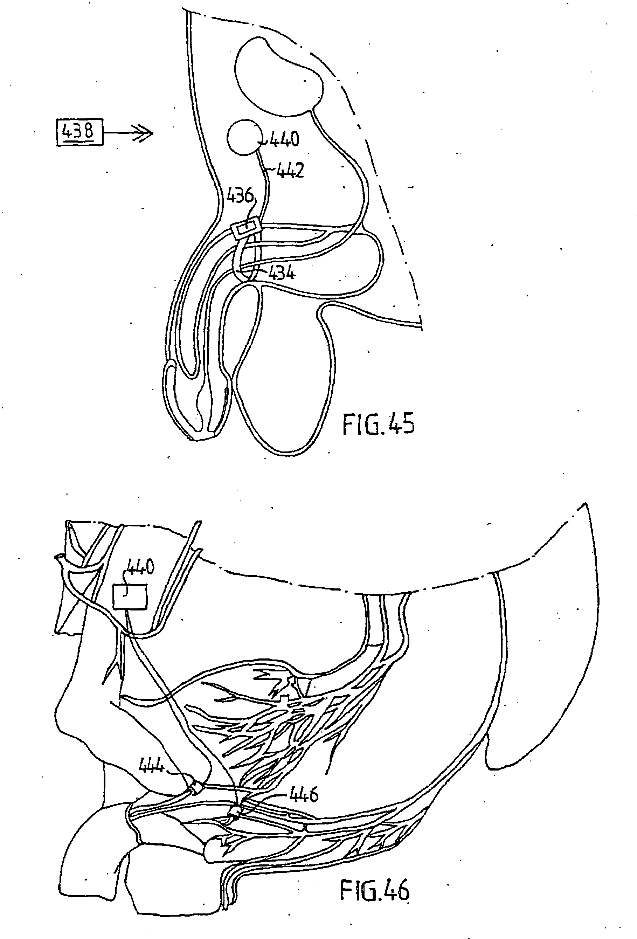

FIGURE 45 schematically illustrates how any of the above-described examples of the male sexual impotence treatment apparatus of the invention may be implanted in a patient. Thus, the apparatus comprises an adjustable restriction device 434 extending around the corpus cavernosum of the patient and a motor operated adjustment device 436 for mechanically adjusting the restriction device 434 to squeeze the corpus cavernosum to thereby restrict the blood flow leaving the penis. The motor, not shown, is integrated in the adjustment device 436 and is reversible to operate the adjustment device 436 to release the corpus cavernosum from the restriction device 434. A wireless remote control of the apparatus comprises an external signal transmitter 438 incorporated in a portable remote-control case and an implanted signal receiver 440, which comprises a control unit for controlling the adjustment device 436 in response to a control signal, for example an electromagnetic wave signal, from the transmitter 438. The signal receiver 440 further comprises an energiser unit which transfers energy from the control signal transmitted by the transmitter 438 into electric energy for energy consuming implanted components of the apparatus, such as the motor for operating the adjustment device 436. The electric energy is conducted via an implanted conductor 442 from the signal receiver 440 to the motor. When the patient wishes to achieve erection, he readily uses the portable remote-control case to activate the implanted adjustment device 436 to temporarily adjust the implanted restriction device 434 to restrict the blood flow leaving his penis.

FIGURE 46 shows an example which is identical to the embodiment of FIGURE 45, except that the restriction device comprises two restriction members 444 and 446 extending around respective exit veins from the penis. A motor operated adjustment device, not shown, is incorporated in each restriction member 444,446.

There are a number of other conceivable alternative embodiments of the invention that give the same result as the above-described embodiments. For example, the microprocessor of the external and implanted, respectively, control unit may be replaced by discrete components. The power amplifier of the external control unit may be omitted if the signals generated by the signal generator are strong enough. Therefore the invention is to be accorded the broadest interpretation of the appended claims to encompass all equivalent structures and assemblies.

## Claims

1. A male sexual impotence treatment apparatus, comprising
an adjustable restriction device (2; 48; 60, 62; 88; 110; 122; 126, 128; 130; 434; 444, 446) implantable in a male impotent patient for directly engaging a portion of the normal penile tissue or the prolongation thereof of the patient, and
an operable adjustment device (12;52;66;90,92;104;110;202;436) implantable in the patient for adjusting the restriction device to temporarily contract said portion of the normal penile tissue or the prolongation thereof to restrict the blood flow leaving the penis, when the patient desires to achieve erection, **characterised in that** the adjustment device comprises
a hydraulic means for operating the adjustment device, comprising:
a first adjustable reservoir (216) containing hydraulic fluid,
a second adjustable reservoir (214) operatively connected to the first reservoir such that changes in the amount of fluid in the second reservoir cause larger changes in the amount of fluid in the first reservoir, and corresponding changes in restriction of the penile tissue or the prolongation thereof,
a third reservoir (210) adapted for subcutaneous implantation, said third reservoir being smaller than the first reservoir, and
a hydraulic tube hydraulically interconnecting the second and third reservoirs, separating the second and third reservoirs from each other, wherein
the third reservoir is adjustable to change the amount of fluid therein to cause fluid flow in the hydraulic tube and a change of amount of fluid in the second reservoir.

2. An apparatus according to claim 1, wherein the restriction device is operable to contract said portion of the patient's penile tissue or the prolongation thereof, when the patient desires to achieve erection, such that the penile arterial blood flow is at least substantially unrestricted, whereas the penile venous blood flow is substantially restricted.

3. An apparatus according to claim 1 or 2, wherein the restriction device is designed for engagement with both of the corpora cavernosa or crura of the penis or the prolongations thereof as a single unit, or for engagement with each of the two corpora cavernosa or crura or the prolongation thereof as two separate units.

4. An apparatus according to claim 1 or 2, wherein said portion of the penile tissue comprises one or more of the exit veins from the penis.

5. An apparatus according to any of claims 1-4, wherein the adjustment device (12;52;66;90,92;104;110;436) is adapted to adjust the restriction device in a non-magnetic or non-thermal manner.

6. An apparatus according to any of claims 1-5, wherein the adjustment device is powered.

7. An apparatus according to any of claims 1-6, wherein the restriction device is non-inflatable.

8. An apparatus according to any of claims 1-7, wherein the restriction device comprises an element to be placed on one side of said portion of the penile tissue, and the adjustment device is adapted to squeeze said portion of the penile tissue between the element and the human bone or tissue to decrease the blood flow leaving the penis.

9. An apparatus according to any one of claims 1-7, wherein the restriction device comprises at least one elongated restriction member (2;48;60,62;88) and forming means (10;94;106;108;118;132) for forming the restriction member into at least a substantially closed loop around said portion of the penile tissue, the loop defining a restriction opening, whereby the adjustment device is adapted to adjust the restriction member in the loop to change the size of the restriction opening.

10. An apparatus according to claim 9, wherein the restriction device comprises several elongated restriction members (2;48;60,62;88) to be formed into at least substantially closed loops around the penile tissue.

11. An apparatus according to claim 9 or 10, wherein, the adjustment device (12;52;66;90,92) is adapted to adjust the longitudinal extension of the elongated restriction member in said loop to change the size of the restriction opening.

12. An apparatus according to claim 9, wherein the restriction member (88;110;122) forms a radial innermost circumferential confinement surface in said loop of the restriction member, and the adjustment device (104;112) is adapted to mechanically adjust the restriction member such that at least a portion of the confinement surface is substantially radially displaced in said loop.

13. An apparatus according to claim 12, wherein the restriction member comprises an elastic annular element (110) forming the confinement surface, and the adjustment device (112) is adapted to change the diameter of the elastic annular element.

14. An apparatus according to claim 9, wherein the forming means (10;94;106;108;118;132) is adapted to form the restriction member (2;48;60,62;88;110;122;126,128;130) into a loop having a predetermined size or a size selected from several predetermined sizes.

15. An apparatus according to claim 9, wherein the adjustment device (12;52;66;90;104;110) is adapted to change the size of the restriction opening (3) such that the outer circumferential confinement surface of the restriction member (2; 48; 60, 62; 88; 110; 122; 126, 128; 130) is changed.

16. An apparatus according to claim 9, wherein the adjustment device (12;52;66;90;104;110;434) is adapted to change the size of the restriction opening (3) such that the outer circumferential confinement surface of the restriction member (2; 48; 60, 62; 88; 110; 122; 126, 128; 130:436) is unchanged.

17. An apparatus according to any one of claims 1-7, wherein the restriction device (412) comprises at least two elements (414) to be placed on different sides of said portion of the penile tissue (406), and the adjustment device is adapted to squeeze said portion of the penile tissue (406) between the elements to restrict the blood flow leaving the penis, and to release said portion of the penile tissue (406) from the elements to increase the blood flow leaving the penis (Figs. 37A,37B).

18. An apparatus according to any one of claims 1-7 wherein the restriction device (418) comprises at least two articulated clamping elements (420) to be positioned on opposite or different sides of said portion of the penile tissue (406), and the adjustment device (422) is adapted to turn the clamping elements toward each other to clamp said portion of the penile tissue (406) between the clamping elements to restrict the blood flow leaving the penis, and to turn the clamping elements away from each other to release said portion of the penile tissue (406) from the clamping elements to increase the blood flow leaving the penis (Fig. 38).

19. An apparatus according to any one of claims 1-7, wherein the restriction device is adapted to bend a portion of said portion of the penile tissue (406) (Figs. 39A-44B).

20. An apparatus according to claim 19, wherein the restriction device (424) comprises at least two bending members (426-430) to be positioned on opposite or different sides of said portion of the penile tissue (406) and to be displaced relative to each other along the penile tissue, and the adjustment device (432) is adapted to move the bending members against said portion of the penile tissue (406) to bend it to restrict the blood flow leaving the penis, and to move the bending members away from said portion of the penile tissue (406) to release it from the bending members to increase the blood flow leaving the penis (Figs. 39A-39C).

21. An apparatus according to claim 20, wherein the bending members comprise rollers.

22. An apparatus according to any one of claims 1-7, wherein the restriction device is adapted to rotate a portion of the penile tissue.

23. An apparatus according to any of claims 1 to 22, further comprising an operation device for transporting fluid between the third and the second reservoirs.

24. An apparatus according to claim 23, wherein the operation device comprises a pump (212).

25. An apparatus according to claim 23 or 24, wherein the operation device is manually operated.

26. An apparatus according to claim 23 or 24, wherein the operation device comprises a motor, preferably an electric motor.

27. An apparatus according to claim 26, wherein the motor is reversible.

28. An apparatus according to claim 26 or 27, comprising an implantable reversing device for reversing the motor.

29. An apparatus according to claims1 to 28, wherein the hydraulic means is devoid of any non-return valve.

30. An apparatus according to any of claims 23-29, wherein the operation device is adapted to operate the adjustment device (5) by using the hydraulic fluid of the first reservoir (216).

31. An apparatus according to claim 30, wherein the reservoir (216) contains a predetermined amount of hydraulic fluid.

32. An apparatus according to claim 3fl, wherein the reservoir (216) comprises first and second wall portions and is adapted to provide relative displacement between the first and second wall portions of the reservoir, in order to change the volume of the reservoir.

33. An apparatus according to claim 32, wherein said relative displacement is performed in response to the pressure in the reservoir.

34. An apparatus according to claim 33, further comprising an alarm adapted to generate an alarm signal in response to the lapse of a predetermined time period during which the pressure controlling in the third reservoir (16) exceeds a predetermined high value.

35. An apparatus according to any of claims 23-34, wherein the operation device comprises magnetic means, electric means or manual manipulation means or a combination thereof.

36. An apparatus according to any of claims 1 to 35, wherein the second reservoir (214) defines a chamber containing servo fluid, and comprises first and second wall portions of the second reservoir, which are displaceable relative to each other to change the volume of the chamber of the second reservoir.

37. An apparatus according to claim 36, wherein the third reservoir (210) defines a chamber for a further predetermined amount of fluid and the hydraulic operation device is adapted to change the volume of the chamber and thereby control the amount of fluid in the second reservoir (214).

38. An apparatus according to claim 37, wherein the third reservoir (210) comprises first and second wall portions, which are displaceable relative to each other to change the volume of the chamber of the third reservoir.

39. An apparatus according to claim 38, wherein the third reservoir (210) increases the amount of fluid in the second reservoir (14) in response to a predetermined first displacement of the first wall portion of the third reservoir relative to the second wall portion of the third reservoir and decreases the amount of fluid in the second reservoir in response to a predetermined second displacement of the first wall portion of the fluid third reservoir to the second wall portion of the third reservoir.

40. An apparatus according to any of claims 1 to 39, wherein the hydraulic means comprises a first reservoir (216) implantable in the patient and containing a predetermined amount of hydraulic fluid, and a conduit providing fluid connection between the first reservoir and the restriction device (2), and operates by distributing hydraulic fluid through the conduit between the first reservoir and the restriction device, the conduit and restriction adjustment device being devoid of any non-return valve to permit free flow of hydraulic fluid in both directions in the conduit.

41. An apparatus according to claim 40, wherein the first reservoir (216) forms a fluid chamber with a variable volume, and the adjustment device is adapted to distribute fluid from the chamber to the restriction device (2) by reduction of the volume of the chamber and to withdraw fluid from the restriction device by expansion of the volume of the chamber.

42. An apparatus according to any of claims 1 to 41, further comprising an injection port subcutaneously implantable in the patient and in fluid communication with the third reservoir (210).

43. An apparatus according to claim 42, wherein the injection port is integrated in the third reservoir (210).

44. An apparatus according to any one of the preceding claims, further comprising a wireless remote control (22,326,332-344) for non-invasively controlling the adjustment device.

45. An apparatus according to claim 44, wherein the remote control (22,326,332-344) comprises a separate signal transmitter and/or receiver (332,336) and an implantable signal receiver and/or transmitter (334,338), for transmitting and/or receiving a control signal.

46. An apparatus according to claim 45, wherein the signal receiver (334,338) comprises a control unit (338) adapted to control the adjustment device (12;52;66;90,92;104;110) in response to the control signal.

47. An apparatus according to claim 46, further comprising an implantable energiser unit (336) for providing energy to energy consuming components of the apparatus to be implanted in the patient.

48. An apparatus according to any of claims 23-47, wherein the operation device comprises an implantable motor or pump.

49. An apparatus according to claims 47 and 48, wherein the control unit (338) is adapted to control the energiser unit (336) to power the motor or pump with energy in response to the control signal.

50. An apparatus according to claim 48 or 49, wherein the motor is an electric motor.

51. An apparatus according to claim 50, comprising a wireless energy transmitter and a and energiser unit, wherein the energiser unit (326), comprising an energy transfer means adapted to transform energy from the wireless energy into electric energy.

52. An apparatus according to claim 47 or 51, wherein the energiser unit (26) is adapted to transform energy from the wireless energy into a direct or alternating current.

53. An apparatus according to claim 51, wherein the energiser unit (326) comprises a rechargeable electric power supply (58) for storing the electric energy and the control unit (338) is adapted to power the electric motor with energy from the rechargeable electric power supply in response to the control signal.

54. An apparatus according to claim 47, wherein the energiser unit (326) comprises a battery, an electrically operable switch adapted to connect the battery to the signal receiver in (334,338) an "on" mode when the switch is powered and to keep the battery disconnected from the signal receiver in a "standby" mode when the switch is not powered, and a rechargeable electric power supply for powering the switch.

55. An apparatus according to claim 54, wherein the control unit (338) is adapted to power the electric motor or pump with energy from the battery in response to a control signal received from the signal transmitter (332,336), when the switch is in its "on" mode.

56. An apparatus according to claim 47, further comprising an external energy transmitter for transmitting wireless energy, wherein the energiser unit comprises a battery and a switch operable by the wireless energy transmitted by the external transmitter, for connecting the battery to the signal receiver in an "on" mode when the switch is powered by the wireless energy and to keep the battery disconnected from the signal receiver in a "standby" mode when the switch is not powered.

57. An apparatus according to claim 1, further comprising an implantable energiser unit (336) for providing energy to energy consuming components of the apparatus to be implanted in the patient.

58. An apparatus according to claim 57, further comprising an external energy transmitter for transmitting wireless energy, wherein the energiser unit is adapted to transform the wireless energy into electric energy.

59. An apparatus according to claim 26 and 58, wherein the energiser unit (326) is adapted to power the electric motor or pump with the electric energy transformed from the wireless energy.

60. An apparatus according to claim 57, further comprising an external energy transmitter for transmitting wireless energy, wherein the energiser unit comprises a battery and a switch operable by the wireless energy transmitted by the external transmitter, for connecting the battery to the implantable energy consuming components of the apparatus in an "on" mode when the switch is powered by the wireless energy and to keep the battery disconnected from the energy consuming components in a "standby" mode when the switch is not powered.

61. An apparatus according to claim 56 or 60, wherein the external energy transmitter is adapted to directly power the switch with the wireless energy to switch into the "on" mode.

62. An apparatus according to claim 44, wherein the remote control (22,326,332-344) is capable of obtaining information from implantable components of the apparatus and of commanding the adjustment device (12;52;66;90;104;110) to adjust the restriction device (2;48;60,62;88;110;122;126,128;130;434) in response to obtained information.

63. An apparatus according to claim 1, further comprising implantable electrical components including at least one voltage level guard.

64. An apparatus according to claim 1, further comprising implantable electrical components including a single voltage level guard.

65. An apparatus according to claim 63 or 64, wherein the electrical components are devoid of any current detector and/or charge level detector.

66. An apparatus according to any of claims 63-65, further comprising an implantable capacitor or accumulator, wherein the charge or discharge of the capacitor or accumulator is controlled by use of the voltage level guard.

67. An apparatus according to claim 1, further comprising a wireless energy transmitter and an energy transfer means (22,326,332-344) for wireless transfer of wireless energy from outside the patient's body to the adjustment device and/or other energy consuming implantable components of the apparatus.

68. An apparatus according to claim 67, wherein wireless energy transmitter is adapted to intermittently transfer the energy in the form of a train of energy pulses and wherein the energy transfer means transfers the wireless energy into electric energy for direct use in connection with the energising of the energy consuming components of the apparatus.

69. An apparatus according to claim 68, wherein the energy transfer means is adapted to transfer electric energy to a train of electric pulses, and further comprising an implantable capacitor for producing the train of energy pulses.

70. An apparatus according to claim 66 or 69, wherein the capacitor has a capacity less than 0,1 µF.

71. An apparatus according to claim 67 in combination with any of claim 24 or 26, , wherein the energy transfer means is adapted to directly power the motor or pump with transferred energy.

72. An apparatus according to claim 24 or 42, , wherein the energy transmission device is adapted to transmit wireless energy in the form of an magnetic field or electromagnetic waves for direct power of the motor or pump, as the wireless energy is being transmitted.

73. An apparatus according to claim 72, wherein the pump is not a plunger type of pump.

74. An apparatus according to claim 73, wherein the energy transfer means is adapted to transfer wireless energy in the form of electromagnetic waves excluding radio waves.

75. An apparatus according to claims 67 or 68, wherein the energy transferred by the energy transfer means comprises an electric field or a magnetic field.

76. An apparatus according to claims 67 or 68, wherein the energy transferred by the energy transfer means comprises a signal.

77. An apparatus according to claim 45 or 76, wherein the signal comprises analog or digital pulses.

78. An apparatus according to claim 77, wherein the analog or digital pulses comprise a magnetic field or an electric field.

79. An apparatus according to claim 45, 75 or 76, wherein the signal comprises a wave signal.

80. An apparatus according to claim 79, wherein the wave signal comprises an electromagnetic wave signal, a sound wave signal or a carrier wave signal.

81. An apparatus according to any one of the preceding claims, further comprising a pressure sensor for directly or indirectly sensing the pressure against the restriction device.

82. An apparatus according to claim 81, wherein the restriction device is controlled in response to signals from the pressure sensor.

83. An apparatus according to claim 1, comprising a wireless energy transmitter and further comprising an implantable energy transfer means for transferring wireless energy directly or indirectly into energy different than the wireless energy for operation of the restriction device.

84. An apparatus according to claim 83, wherein the energy transfer means transforms the wireless energy into kinetic energy for operation of the restriction device.

85. An apparatus according to claim 84, wherein the energy transfer means transforms the wireless energy in the form of sound waves into electric energy for operation of the restriction device.

86. An apparatus according to any one of the preceding claims, further comprising an implantable reversing device, wherein the restriction device is capable of performing a reversible function and the reversing device reverses the reversible function.

87. An apparatus according to any one of the preceding claims, further comprising an implantable accumulator or battery and means for controlling the accumulator or battery from outside the patient's body to supply energy to the adjustment device and/or other implantable energy consuming components of the apparatus.

88. An apparatus according to claim 1, wherein the adjustment device is adapted to adjust the restriction device in a non-manual manner.

89. An apparatus according to any of the preceding claims, wherein the restriction device is designed for implantation in the base of the patient's penis or its prolongation.

90. An apparatus according to claim 1, further comprising an adjustment device for adjusting the restriction device to change the restriction of the blood flow passageway, wherein the adjustment device is adapted to mechanically adjust the restriction device, or adapted to hydraulically adjust the restriction device by using hydraulic means which is devoid of hydraulic fluid of the kind having a viscosity that substantially increases when exposed to heat or a magnetic field.

91. An apparatus according to claim 90, wherein the energy transforming device transforms the wireless energy in the form of sound waves directly into electric energy.

92. An apparatus according to claim 67, 85 or 91, wherein the energy transfer means comprises a capacitor.

93. An apparatus according to claim 92, wherein the capacitor is adapted to produce electric pulses from the transformed electric energy.

94. An apparatus according to claim 80, wherein the carrier signal is frequency, amplitude or frequency and amplitude modulated.

95. An apparatus according to claim 80, wherein the control signal comprises a wave signal comprising one of a sound wave signal including an ultrasound wave signal, an electromagnetic wave signal including an infrared light signal, a visible light signal, an ultra violet light signal and a laser light signal, a micro wave signal, a radio wave signal, an x-ray radiation signal, and a gamma radiation signal.

96. An apparatus according to claim 67, wherein the energy transfer means is adapted to transfer magnetic energy, non-magnetic energy, electromagnetic energy, non-electromagnetic energy, kinetic energy, non-kinetic energy, sonic energy, non-sonic energy, thermal energy or non-thermal energy.

97. An apparatus according to claim 3 or 4, wherein the restriction device is adapted to control the cross-sectional area of a blood flow passageway formed by the patient's penile exit veins or corpora/crura cavernosa.

98. An apparatus according to claim 97, wherein the restriction device is operable to open and close the blood flow passageway.

99. An apparatus according to claim 98, wherein the restriction device is adapted to steplessly control the cross-sectional area of the blood flow passageway.

100. An apparatus according to claim 67, comprising an energiser unit (326), wherein the transferred energy are stored in the energiser unit for later use for any of the energy consuming implantable components of the apparatus.

101. An apparatus according to claim 100, wherein the energiser unit (326), comprises a rechargeable battery or accumulator for supplying energy to any of the energy consuming implantable components of the apparatus.

102. An apparatus according to claim 48-49, wherein the energiser unit (326) comprising an energy transfer means, wherein the energy transfer means is adapted to transform energy from the control signal, as it is transmitted to the signal receiver (334,338), into electric energy.

## Patentansprüche

1. Vorrichtung zur Behandlung von männlicher sexueller Impotenz, umfassend:
eine einstellbare Verengungsvorrichtung (2, 48, 60, 62, 88, 110, 122, 126, 128, 130, 434, 444, 446), die in einen männlichen Impotenzpatienten implantiert werden kann, um direkt mit einem Teil des normalen penilen Gewebes oder der Verlängerung davon des Patienten in Eingriff zu stehen, und
eine betätigbare Einstellvorrichtung (15, 52, 66, 90, 92, 104, 110, 202, 436), die in den Patienten implantiert werden kann, zum Einstellen der Verengungsvorrichtung, um den Teil des normalen penilen Gewebes oder die Verlängerung davon temporär zusammenzuziehen, so dass der Blutstrom, der den Penis verlässt, verengt wird, wenn der Patient wünscht, Erektion zu erreichen, **dadurch gekennzeichnet, dass** die Einstellvorrichtung umfasst:
eine Hydraulikeinrichtung zum Betätigen der Einstellvorrichtung, umfassend
einen ersten anpassbaren Behälter (216), der Hydraulikfluid enthält,
einen zweiten anpassbaren Behälter (214), derartig mit dem ersten Behälter in Wirkverbindung stehend, dass Änderungen in der Fluidmenge in dem zweiten Behälter größere Änderungen der Fluidmenge in dem ersten Behälter und entsprechende Änderungen in der Verengung des penilen Gewebes oder der Verlängerung davon bewirken,
einen dritten Behälter (210), eingerichtet für subkutane Implantation, wobei der dritte Behälter kleiner als der erste Behälter ist, und
eine Hydraulikrohrleitung, die den zweiten und den dritten Behälter hydraulisch verbindet und den zweiten und den dritten Behälter voneinander separiert, wobei
der dritte Behälter anpassbar ist, die Fluidmenge darin zu ändern, um Fluidströmung in der Hydraulikrohrleitung und eine Änderung der Fluidmenge in dem zweiten Behälter zu bewirken.

2. Vorrichtung nach Anspruch 1, wobei die Verengungsvorrichtung funktionsfähig ist, um den Teil des penilen Gewebes oder der Verlängerung davon des Patienten, wenn der Patient wünscht, Erektion zu erreichen, so zusammenzuziehen, dass der penile arterielle Blutstrom im Wesentlichen unbehindert ist, wohingegen der penile venöse Blutstrom im Wesentlichen verengt ist.

3. Vorrichtung nach Anspruch 1 oder 2, wobei die Verengungsvorrichtung ausgelegt ist, um mit beiden von der Corpora cavernosa oder Crura cavernosa des Penis oder den Verlängerungen davon als eine Einzeleinheit in Eingriff zu stehen oder mit jeder von den zwei Corpora cavernosa oder Crura cavernosa oder der Verlängerung davon als zwei separate Einheiten in Eingriff zu stehen.

4. Vorrichtung nach Anspruch 1 oder 2, wobei der Teil des penilen Gewebes eine oder mehrere der austretenden Venen des Penis umfasst.

5. Vorrichtung nach einem der Ansprüche 1 bis 4, wobei die Einstellvorrichtung (12, 52, 66, 90, 92, 104, 110, 436) eingerichtet ist, um die Verengungsvorrichtung in einer nichtmagnetischen oder nichtthermischen Art und Weise einzustellen.

6. Vorrichtung nach einem der Ansprüche 1 bis 5, wobei die Einstellvorrichtung mit Energie versorgt wird.

7. Vorrichtung nach einem der Ansprüche 1 bis 6, wobei die Verengungsvorrichtung nicht aufblasbar ist.

8. Vorrichtung nach einem der Ansprüche 1 bis 7, wobei die Verengungsvorrichtung ein Element umfasst, das auf einer Seite des Teils des penilen Gewebes anzuordnen ist, und die Einstellvorrichtung eingerichtet ist, um den Teil des penilen Gewebes zwischen dem Element und dem menschlichen Knochen oder dem Gewebe zusammenzudrücken, um den Blutstrom, der den Penis verlässt, zu verringern.

9. Vorrichtung nach einem der Ansprüche 1 bis 7, wobei die Verengungsvorrichtung wenigstens ein längliches Verengungsglied (2, 48, 60, 62, 88) und eine Formeinrichtung (10, 94, 106, 108, 118, 132) zum Formen des Verengungsgliedes in wenigstens eine im Wesentlichen geschlossene Schleife um den Teil des penilen Gewebes umfasst, wobei die Schleife eine Verengungsöffnung bildet, wodurch die Einstellvorrichtung eingerichtet ist, das Verengungsglied in der Schleife einzustellen, um die Größe der Verengungsöffnung zu ändern.

10. Vorrichtung nach Anspruch 9, wobei die Verengungsvorrichtung mehrere längliche Verengungsglieder (2, 48, 60, 62, 88) umfasst, die in wenigstens im Wesentlichen geschlossene Schleifen um das penile Gewebe zu formen sind.

11. Vorrichtung nach Anspruch 9 oder 10, wobei die Einstellvorrichtung (15, 52, 66, 90, 92) eingerichtet ist, um die Längserstreckung des länglichen Verengungsgliedes in der Schleife einzustellen, um die Größe der Verengungsöffnung zu ändern.

12. Vorrichtung nach Anspruch 9, wobei das Verengungsglied (88, 110, 122) eine radiale innerste Begrenzungsfläche in der Schleife des Verengungsgliedes bildet und die Einstellvorrichtung (104, 112) eingerichtet ist, um das Verengungsglied so einzustellen, dass wenigstens ein Teil der Begrenzungsfläche im Wesentlichen radial in der Schleife verschoben wird.

13. Vorrichtung nach Anspruch 12, wobei das Verengungsglied ein elastisches Ringelement (110), das die Begrenzungsfläche bildet, umfasst und die Einstellvorrichtung (112) eingerichtet ist, um den Durchmesser des elastischen Ringelements zu ändern.

14. Vorrichtung nach Anspruch 9, wobei die Formeinrichtung (10, 94, 106, 108, 118, 132) eingerichtet ist, um das Verengungsglied (2, 48, 60, 62, 88, 110, 122, 126, 128, 130) in eine Schleife zu formen, die eine vorgegebene Größe oder eine Größe, die aus mehreren vorgegebenen Größen ausgewählt wird, hat.

15. Vorrichtung nach Anspruch 9, wobei die Einstellvorrichtung (15, 52, 66, 90,104, 110) eingerichtet ist, um die Größe der Verengungsöffnung (3) so zu ändern, dass die Außenumfangs-Begrenzungsfläche des Verengungsgliedes (2, 48, 60, 62, 88, 110, 122, 126, 128, 130) geändert wird.

16. Vorrichtung nach Anspruch 9, wobei die Einstellvorrichtung (15, 52, 66, 90,104, 110, 434) eingerichtet ist, um die Größe der Verengungsöffnung (3) so zu ändern, dass die Außenumfangs-Begrenzungsfläche des Verengungsgliedes (2, 48, 60, 62, 88, 110, 122, 126, 128, 130, 436) gleichbleibend ist.

17. Vorrichtung nach einem der Ansprüche 1 bis 7, wobei die Verengungsvorrichtung (412) wenigstens zwei Elemente umfasst, die auf verschiedenen Seiten des Teils des penilen Gewebes (406) anzuordnen sind, und die Einstellvorrichtung eingerichtet ist, den Teil des penilen Gewebes (406) zwischen den Elementen zusammenzudrücken, so dass der Blutstrom, der den Penis verlässt, verengt wird, und den Teil des penilen Gewebes (406) von den Elementen zu lösen, um den Blutstrom, der den Penis verlässt, zu verstärken (Figuren 37A und 37B).

18. Vorrichtung nach einem der Ansprüche 1 bis 7, wobei die Verengungsvorrichtung (418) wenigstens zwei bewegliche Klemmelemente (420) umfasst, die auf einer oder verschiedenen Seiten des Teils des penilen Gewebes (406) anzuordnen sind, und die Einstellvorrichtung (422) eingerichtet ist, die Klemmelemente zueinander zu drehen, um den Teil des penilen Gewebes (406) zwischen den Klemmelementen einzuklemmen, so dass der Blutstrom, der den Penis verlässt, verengt wird, und die Klemmelemente voneinander wegzudrehen, um den Teil des penilen Gewebes (406) aus den Klemmelementen zu lösen und den Blutstrom, der den Penis verlässt, zu verstärken (Figur 38).

19. Vorrichtung nach einem der Ansprüche 1 bis 7, wobei die Verengungsvorrichtung eingerichtet ist, um einen Teil des penilen Gewebes (406) zu biegen (Figuren 39A bis 44B).

20. Vorrichtung nach Anspruch 19, wobei die Verengungsvorrichtung (424) wenigstens zwei Biegeglieder (426 bis 430) umfasst, die auf gegenüberliegenden oder verschiedenen Seiten des Teils des penilen Gewebes (406) anzuordnen sind, und relativ zueinander entlang des penilen Gewebes zu verschieben sind, und die Einstellvorrichtung (432) eingerichtet ist, die Biegeglieder (406) gegen den Teil des penilen Gewebes zu verschieben, um ihn zu biegen, so dass der Blutstrom, der den Penis verlässt, verengt wird, und um die Biegeglieder von dem Teil des penilen Gewebes (406) wegzubewegen, so dass er von den Biegegliedern freigegeben wird, um den Blutstrom, der den Penis verlässt, zu verstärken (Figuren 39A bis 39C).

21. Vorrichtung nach Anspruch 20, wobei die Biegeglieder Rollen umfassen.

22. Vorrichtung nach einem der Ansprüche 1 bis 7, wobei die Verengungsvorrichtung eingerichtet ist, um einen Teil des penilen Gewebes zu drehen.

23. Vorrichtung nach einem der Ansprüche 1 bis 22, des Weiteren eine Betätigungsvorrichtung zum Transportieren von Fluid zwischen dem dritten und dem zweiten Behälter umfassend.

24. Vorrichtung nach Anspruch 23, wobei die Betätigungsvorrichtung eine Pumpe (212) umfasst.

25. Vorrichtung nach Anspruch 23 oder 24, wobei die Betätigungsvorrichtung manuell betätigt wird.

26. Vorrichtung nach Anspruch 23 oder 24, wobei die Betätigungsvorrichtung einen Motor, bevorzugt einen Elektromotor, umfasst.

27. Vorrichtung nach Anspruch 26, wobei der Motor umkehrbar ist.

28. Vorrichtung nach Anspruch 26 oder 27, eine implantierbare Umkehrvorrichtung zum Umkehren des Motors umfassend.

29. Vorrichtung nach einem der Ansprüche 1 bis 28, wobei die Hydraulikeinrichtung ohne jedes Rückschlagventil ist.

30. Vorrichtung nach einem der Ansprüche 23 bis 29, wobei die Betätigungsvorrichtung eingerichtet ist, um die Einstellvorrichtung (5) mit Hilfe des Hydraulikfluids des ersten Behälters (216) zu betätigen.

31. Vorrichtung nach Anspruch 30, wobei der Behälter (216) eine vorgegebene Menge Hydraulikfluid enthält.

32. Vorrichtung nach Anspruch 31, wobei der Behälter (216) erste und zweite Wandteile umfasst und eingerichtet ist, um Relativverschiebung zwischen dem ersten und dem zweiten Wandteil des Behälters bereitzustellen, um das Volumen des Behälters zu ändern.

33. Vorrichtung nach Anspruch 32, wobei die Relativverschiebung in Reaktion auf den Druck in dem Behälter durchgeführt wird.

34. Vorrichtung nach Anspruch 33, des Weiteren eine Warneinrichtung umfassend, die eingerichtet ist, um in Reaktion auf das Verstreichen eines vorgegebenen Zeitraums, in dem die Druckregelung in dem dritten Behälter (16) einen vorgegebenen Höchstwert übersteigt, ein Alarmsignal erzeugt.

35. Vorrichtung nach einem der Ansprüche 23 bis 34, wobei die Betätigungsvorrichtung Magneteinrichtungen, elektrische Einrichtungen oder manuelle Manipulationseinrichtungen oder eine Kombination davon umfasst.

36. Vorrichtung nach einem der Ansprüche 1 bis 35, wobei der zweite Behälter (214) eine Kammer bildet, die Servofluid enthält und einen ersten und einen zweiten Wandabschnitt des zweiten Behälters umfasst, die relativ zueinander verschiebbar sind, um das Volumen der Kammer des zweiten Behälters zu ändern.

37. Vorrichtung nach Anspruch 36, wobei der dritte Behälter (210) eine Kammer für eine weitere vorgegebene Menge von Fluid umfasst und die hydraulische Betätigungsvorrichtung eingerichtet ist, um das Volumen der Kammer zu ändern und **dadurch** die Fluidmenge in dem zweiten Behälter (214) zu regeln.

38. Vorrichtung nach Anspruch 37, wobei der dritte Behälter (210) einen ersten und einen zweiten Wandteil umfasst, die relativ zueinander verschiebbar sind, um das Volumen der Kammer des dritten Behälters zu ändern.

39. Vorrichtung nach Anspruch 38, wobei der dritte Behälter (210) die Fluidmenge in dem zweiten Behälter (14) in Reaktion auf eine vorgegebene erste Verschiebung des ersten Wandteils des dritten Behälters relativ zu dem zweiten Wandteil des dritten Behälters vergrößert und in Reaktion auf eine vorgegebene zweite Verschiebung des ersten Wandteils des dritten Fluidbehälters zu dem zweiten Wandteil des dritten Behälters die Fluidmenge in dem zweiten Behälter verkleinert.

40. Vorrichtung nach einem der Ansprüche 1 bis 39, wobei die Hydraulikeinrichtung einen ersten Behälter (216), der in den Patienten implantierbar ist und eine vorgegebene Menge Hydraulikflüssigkeit enthält, und eine Leitung, die Fluidverbindung zwischen dem ersten Behälter und der Verengungsvorrichtung (2) bereitstellt, umfasst und durch Verteilen von Hydraulikfluid durch die Leitung zwischen dem ersten Behälter und der Verengungsvorrichtung arbeitet, wobei die Leitung und die Verengungs-Einstellvorrichtung ohne jedes Rückschlagventil sind, um freies Strömen von Hydraulikfluid in beide Richtungen in der Leitung bereitzustellen.

41. Vorrichtung nach Anspruch 40, wobei der erste Behälter (216) eine Fluidkammer mit einem variablen Volumen bildet und die Einstellvorrichtung eingerichtet ist, um durch Reduzieren des Volumens der Kammer Fluid von der Kammer zu der Verengungsvorrichtung (2) zu verteilen und um der Verengungsvorrichtung durch Vergrößerung des Volumens der Kammer Fluid zu entziehen.

42. Vorrichtung nach einem der Ansprüche 1 bis 41, des Weiteren einen Injektionsport, subkutan in den Patienten implantierbar und in Fluidkommunikation mit dem dritten Behälter (210), umfassend.

43. Vorrichtung nach Anspruch 42, wobei der Injektionsport in den dritten Behälter (210) integriert ist.

44. Vorrichtung nach einem der vorhergehenden Ansprüche, des Weiteren eine drahtlose Fernbedienung (22, 326, 332 bis 344) zum nichtinvasiven Steuern der Einstellvorrichtung umfassend.

45. Vorrichtung nach Anspruch 44, wobei die Fernbedienung (22, 326, 332 bis 344) einen separaten Signalsender und/oder -empfänger (332, 336) und einen implantierbaren Signalempfänger und/oder -sender (334, 338) zum Senden und/oder Empfangen eines Steuersignals umfasst.

46. Vorrichtung nach Anspruch 45, wobei der Signalempfänger (334, 338) eine Steuereinheit (338) umfasst, die eingerichtet ist, um die Einstellvorrichtung (15, 52, 66, 90, 92, 104, 110) in Reaktion auf das Steuersignal zu steuern.

47. Vorrichtung nach Anspruch 46, des Weiteren eine implantierbare Energizer-Einheit (336) zum Bereitstellen von Energie für Energie verbrauchende Komponenten der Vorrichtung, die in den Patienten zu implantieren sind, umfassend.

48. Vorrichtung nach einem der Ansprüche 23 bis 47, wobei die Betätigungsvorrichtung einen implantierbaren Motor oder eine implantierbare Pumpe umfasst.

49. Vorrichtung nach den Ansprüchen 47 und 48, wobei die Steuereinheit (338) eingerichtet ist, um die Energizer-Einheit (336) so zu steuern, dass sie den Motor oder die Pumpe in Reaktion auf das Steuersignal mit Energie versorgt.

50. Vorrichtung nach den Anspruch 48 oder 49, wobei der Motor ein Elektromotor ist.

51. Vorrichtung nach Anspruch 50, einen drahtlosen Energietransmitter und eine Energizer-Einheit umfassend, wobei die Energizer-Einheit (326) eine Energieübertragungseinrichtung umfasst, die geeignet ist, Energie von der drahtlosen Energie in elektrische Energie umzuwandeln.

52. Vorrichtung nach Anspruch 47 oder 51, wobei die Energizer-Einheit (26) eingerichtet ist, um Energie von der drahtlosen Energie in einen Gleich- oder Wechselstrom umzuwandeln.

53. Vorrichtung nach Anspruch 51, wobei die Energizer-Einheit (326) eine wiederaufladbare elektrische Leistungsversorgung (58) zum Speichern der elektrischen Energie umfasst und die Steuereinheit (338) eingerichtet ist, um den Elektromotor in Reaktion auf das Steuersignal mit Energie aus der wiederaufladbaren elektrischen Leistungsversorgung zu versorgen.

54. Vorrichtung nach Anspruch 47, wobei die Energizer-Einheit (326) eine Batterie, einen elektrisch betätigbaren Schalter, eingerichtet, um die Batterie mit dem Signalempfänger (334, 338) in einem Modus EIN zu verbinden, wenn der Schalter mit Energie versorgt wird, und die Batterie getrennt von dem Signalempfänger in einem Modus STANDBY zu halten, wenn der Schalter nicht mit Energie versorgt wird, und eine wiederaufladbare elektrische Leistungsversorgung zum Versorgen des Schalters mit Energie umfasst.

55. Vorrichtung nach Anspruch 54, wobei die Steuereinheit (338) eingerichtet ist, um den Elektromotor oder die Elektropumpe in Reaktion auf ein von dem Signalsender (332, 336) empfangenes Steuersignal mit Energie zu versorgen, wenn der Schalter in seinem Modus EIN ist.

56. Vorrichtung nach Anspruch 47, des Weiteren einen externen Energietransmitter zum Übertragen drahtloser Energie umfassend, wobei die Energizer-Einheit eine Batterie und einen Schalter umfasst, der durch die drahtlose Energie, die durch den externen Transmitter übertragen wird, betätigen kann, um die Batterie mit dem Signalempfänger in einem Modus EIN zu verbinden, wenn der Schalter mit der drahtlosen Energie versorgt wird, und die Batterie getrennt von dem Signalempfänger in einem Modus STANDBY zu halten, wenn der Schalter nicht mit Energie versorgt wird.

57. Vorrichtung nach Anspruch 1, des Weiteren eine implantierbare Energizer-Einheit (336) zum Bereitstellen von Energie für Energie verbrauchende Komponenten der Vorrichtung, die in den Patienten zu implantieren sind, umfassend.

58. Vorrichtung nach Anspruch 57, des Weiteren einen externen Energietransmitter zum Übertragen von drahtloser Energie umfassend, wobei die Energizer-Einheit eingerichtet ist, um die drahtlose Energie in elektrische Energie umzuwandeln.

59. Vorrichtung nach Anspruch 26 und 58, wobei die Energizer-Einheit (326) eingerichtet ist, um den Elektromotor oder die Elektropumpe mit der aus der drahtlosen Energie umgewandelten elektrischen Energie zu versorgen.

60. Vorrichtung nach Anspruch 57, des Weiteren einen externen Energietransmitter zum Übertragen von drahtloser Energie umfassend, wobei die Energizer-Einheit eine Batterie und einen Schalter umfasst, der durch die durch den externen Transmitter übertragene drahtlose Energie betätigt werden kann, um die Batterie mit den implantierbaren Energie verbrauchenden Komponenten der Vorrichtung in einem Modus EIN zu verbinden, wenn der Schalter mit der drahtlosen Energie versorgt wird, und die Batterie getrennt von dem Signalempfänger in einem Modus STANDBY zu halten, wenn der Schalter nicht mit Energie versorgt wird.

61. Vorrichtung nach Anspruch 56 oder 60, wobei der externe Energietransmitter eingerichtet ist, den Schalter direkt mit der drahtlosen Energie zu versorgen, um in den Modus EIN zu schalten.

62. Vorrichtung nach Anspruch 44, wobei die Fernbedienung (22, 326, 332 bis 344) fähig ist, von implantierbaren Komponenten der Vorrichtung Informationen zu beziehen und die Einstellvorrichtung (15, 52, 66, 90, 104, 110) zu befehligen, um die Verengungsvorrichtung (2, 48, 60, 62, 88, 110, 122, 126, 128, 130, 434) in Reaktion auf die erhaltenen Informationen zu regeln.

63. Vorrichtung nach Anspruch 1, des Weiteren implantierbare elektrische Komponenten, einschließlich wenigstens eines Spannungsschutzes, umfassend.

64. Vorrichtung nach Anspruch 1, des Weiteren implantierbare elektrische Komponenten, einschließlich eines einzelnen Spannungsschutzes, umfassend.

65. Vorrichtung nach Anspruch 63 oder 64, wobei die elektrischen Komponenten ohne jeden Stromdetektor und/oder Ladezustandsdetektor sind.

66. Vorrichtung nach einem der Ansprüche 63 bis 65, des Weiteren einen implantierbaren Kondensator oder Akkumulator umfassend, wobei das Laden oder Entladen des Kondensators oder Akkumulators mit Hilfe des Spannungsschutzes gesteuert wird.

67. Vorrichtung nach Anspruch 1, des Weiteren einen drahtlosen Energietransmitter und eine Energieübertragungseinrichtung (22, 326, 332 bis 344) zum drahtlosen Übertragen von drahtloser Energie von außerhalb des Körpers des Patienten zu der Einstellvorrichtung und/oder anderen Energie verbrauchenden implantierbaren Komponenten der Vorrichtung umfassend.

68. Vorrichtung nach Anspruch 67, wobei der drahtlose Energietransmitter eingerichtet ist, um die Energie intermittierend in der Form einer Reihe von Energieimpulsen zu übertragen, und wobei die Energieübertragungseinrichtung die drahtlose Energie in elektrische Energie zur direkten Nutzung in Verbindung mit dem Erregen der Energie verbrauchenden Komponenten der Vorrichtung überträgt.

69. Vorrichtung nach Anspruch 68, wobei die Energieübertragungseinrichtung eingerichtet ist, um elektrische Energie in einer Reihe von elektrischen Impulsen zu übertragen und des Weiteren einen implantierbaren Kondensator zum Erzeugen der Reihe von Energieimpulsen umfasst.

70. Vorrichtung nach Anspruch 66 oder 69, wobei der Kondensator eine Kapazität von weniger als 0,1 µF hat.

71. Vorrichtung nach Anspruch 67 in Verbindung mit einem der Ansprüche 24 oder 26, wobei die Energieübertragungseinrichtung eingerichtet ist, um den Motor oder die Pumpe direkt mit übertragener Energie zu versorgen.

72. Vorrichtung nach Anspruch 24 oder 42, wobei die Energieübertragungseinrichtung eingerichtet ist, um drahtlose Energie in der Form eines Magnetfeldes oder elektromagnetischer Wellen zum direkten Versorgen des Motors oder der Pumpe mit Energie, während die Energie übertragen wird, zu übertragen.

73. Vorrichtung nach Anspruch 72, wobei die Pumpe keine Pumpe des Kolbentyps ist.

74. Vorrichtung nach Anspruch 73, wobei die Energieübertragungseinrichtung eingerichtet ist, um die drahtlose Energie in Form von elektromagnetischen Wellen, ausgenommen Funkwellen, zu übertragen.

75. Vorrichtung nach Anspruch 67 oder 68, wobei die durch die Energieübertragungseinrichtung übertragene Energie ein elektrisches Feld oder ein Magnetfeld umfasst.

76. Vorrichtung nach Anspruch 67 oder 68, wobei die durch die Energieübertragungseinrichtung übertragene Energie ein Signal umfasst.

77. Vorrichtung nach Anspruch 45 oder 76, wobei das Signal analoge oder digitale Impulse umfasst.

78. Vorrichtung nach Anspruch 77, wobei die analogen oder digitalen Impulse ein Magnetfeld oder ein elektrisches Feld umfassen.

79. Vorrichtung nach Anspruch 45, 75 oder 76, wobei das Signal ein Wellensignal umfasst.

80. Vorrichtung nach Anspruch 79, wobei das Wellensignal ein elektromagnetisches Wellensignal, ein Schallwellensignal oder ein Trägerwellensignal umfasst.

81. Vorrichtung nach e!nem der vorhergehenden Ansprüche, des Weiteren e!nen Drucksensor zum direkten oder indirekten Erfassen des Drucks an der Verengungsvorrichtung umfassend.

82. Vorrichtung nach Anspruch 81, wobei die Verengungsvorrichtung in Reaktion auf Signale von dem Drucksensor geregelt wird.

83. Vorrichtung nach Anspruch 1, einen drahtlosen Energietransmitter umfassend und des Weiteren eine implantierbare Energieübertragungseinrichtung zum Übertragen von drahtloser Energie direkt oder indirekt in Energie, die von der drahtlosen Energie verschieden ist, zum Betreiben der Verengungsvorrichtung umfassend.

84. Vorrichtung nach Anspruch 83, wobei die Energieübertragungseinrichtung die drahtlose Energie in kinetische Energie zum Betreiben der Verengungsvorrichtung transformiert.

85. Vorrichtung nach Anspruch 84, wobei die Energieübertragungseinrichtung die drahtlose Energie in der Form von Schallwellen in elektrische Energie zum Betätigen der Verengungsvorrichtung transformiert.

86. Vorrichtung nach einem der vorhergehenden Ansprüche, die des Weiteren eine implantierbare Umkehreinrichtung umfasst, wobei die Verengungsvorrichtung in der Lage ist, eine umkehrbare Funktion auszuführen und die Umkehreinrichtung die umkehrbare Funktion umkehrt.

87. Vorrichtung nach einem der vorhergehenden Ansprüche, die des Weiteren einen implantierbaren Akkumulator oder eine implantierbare Batterie und Einrichtungen zum Steuern des Akkumulators oder der Batterie von außerhalb des Körpers des Patienten umfasst, um der Einstellvorrichtung und/oder anderen implantierbaren Energie verbrauchenden Komponenten der Vorrichtung Energie zuzuführen.

88. Vorrichtung nach Anspruch 1, wobei die Einstellvorrichtung eingerichtet ist, um die Verengungsvorrichtung in einer nicht manuellen Art und Weise einzustellen.

89. Vorrichtung nach einem der vorhergehenden Ansprüche, wobei die Verengungsvorrichtung ausgelegt ist, um in die Basis des Penis oder ihrer Verlängerung des Patienten implantiert zu werden.

90. Vorrichtung nach Anspruch 1, die des Weiteren eine Einstellvorrichtung zum Einstellen der Verengungsvorrichtung, die Verengung des Blutstromdurchgangsweges zu ändern, umfasst, wobei die Einstellvorrichtung eingerichtet ist, um die Verengungsvorrichtung mechanisch einzustellen, oder eingerichtet ist, um die Verengungsvorrichtung mit Hilfe einer Hydraulikeinrichtung einzustellen, die ohne Hydraulikfluid der Art ist, das eine Viskosität hat, die sich wesentlich erhöht, wenn es Hitze oder einem Magnetfeld ausgesetzt wird.

91. Vorrichtung nach Anspruch 90, wobei die Energieumwandlungsvorrichtung die drahtlose Energie in der Form von Schallwellen direkt in elektrische Energie umwandelt.

92. Vorrichtung nach Anspruch 67, 85 oder 91, wobei die Energieübertragungseinrichtung einen Kondensator umfasst.

93. Vorrichtung nach Anspruch 92, wobei der Kondensator eingerichtet ist, um aus der umgewandelten elektrischen Energie elektrische Impulse zu erzeugen.

94. Vorrichtung nach Anspruch 80, wobei das Trägersignal frequenz-, amplituden- oder frequenz- und amplitudenmoduliert ist.

95. Vorrichtung nach Anspruch 80, wobei das Steuersignal ein Wellensignal umfasst, bestehend aus einem von einem Schallwellensignal, einschließlich eines Ultraschallwellensignals, einem elektromagnetischen Wellensignal, einschließlich eines IR-Lichtsignals, eines sichtbaren Lichtsignals, eines Ultraviolett-Lichtsignals und eines Laserlichtsignals, einem Mikrowellensignal, einem Funkwellensignal, einem Röntgenstrahlungssignal und einem Gammastrahlungssignal.

96. Vorrichtung nach Anspruch 67, wobei die Energieübertragungseinrichtung eingerichtet ist, um magnetische Energie, nicht magnetische Energie, elektromagnetische Energie, nicht elektromagnetische Energie, kinetische Energie, nicht kinetische Energie, Schallenergie, Nichtschall-Energie, thermische Energie oder nicht thermische Energie zu übertragen.

97. Vorrichtung nach Anspruch 3 oder 4, wobei die Verengungsvorrichtung eingerichtet ist, um die Querschnittsfläche eines durch die penilen austretenden Venen des Patienten oder der Corpora/Crura cavernosa gebildeten Blutstromdurchgangsweges zu regeln.

98. Vorrichtung nach Anspruch 97, wobei die Verengungsvorrichtung betätigt werden kann, um den Blutstromdurchgangsweg zu öffnen oder zu schließen.

99. Vorrichtung nach Anspruch 98, wobei die Verengungsvorrichtung eingerichtet ist, um die Querschnittsfläche des Blutstromdurchgangsweges stufenlos zu regeln.

100. Vorrichtung nach Anspruch 67, eine Energizer-Einheit (326) umfassend, wobei die übertragenen Energien zur späteren Nutzung für jede der Energie verbrauchenden implantierbaren Komponenten der Vorrichtung in der Energizer-Einheit gespeichert werden.

101. Vorrichtung nach Anspruch 100, wobei die Energizer-Einheit (326) eine wiederaufladbare Batterie oder einen Akkumulator zum Zuführen von Energie zu jeder der Energie verbrauchenden implantierbaren Komponenten der Vorrichtung umfasst.

102. Vorrichtung nach Anspruch 48 und 49, wobei die Energizer-Einheit (326) eine Energieübertragungseinrichtung umfasst, wobei die Energieübertragungseinrichtung eingerichtet ist, um Energie aus dem Steuersignal, während es zu dem Signalempfänger (334, 338) gesendet wird, in elektrische Energie zu umzuwandeln.

## Revendications

1. Appareil de traitement de l'impuissance sexuelle masculine, comprenant
un dispositif de restriction réglable (2 ; 48 ; 60, 62 ; 88 ; 110 ; 122 ; 126 ; 128 ; 130 ; 434 ; 444,446) pouvant être implanté chez un patient impuissant masculin afin de directement engager une partie du tissu pénien normal ou le prolongement de celui-ci du patient, et
un dispositif de réglage opérable (12 ; 52 ; 66 ; 90, 92 ; 104 ; 110 ; 202 ; 436) pouvant être implanté chez le patient pour régler le dispositif de restriction afin de contracter temporairement ladite partie du tissu pénien normal ou le prolongement de celui-ci afin de retreindre le flux sanguin quittant le pénis, lorsque le patient désire avoir une érection, **caractérisé en ce que** le dispositif de réglage comprend
un moyen hydraulique pour opérer le dispositif de réglage, comprenant :
un premier réservoir réglable (216) contenant un fluide hydraulique,
un deuxième réservoir réglable (214) relié de manière opérationnelle au premier réservoir de sorte que les modifications de la quantité de fluide dans le deuxième réservoir causent des modifications plus importantes de la quantité de fluide dans le premier réservoir, et les modifications correspondantes dans la restriction du tissu pénien ou le prolongement de celui-ci
un troisième réservoir (210) adapté à une implantation sous-cutanée, ledit troisième réservoir étant plus petit que le premier réservoir, et
un tube hydraulique interconnectant hydrauliquement les deuxième et troisième réservoirs, séparant les deuxième et troisième réservoirs l'un de l'autre, dans lequel
le troisième réservoir est réglable afin de modifier la quantité de fluide dans celui-ci pour causer la circulation du fluide dans le tube hydraulique et une modification de la quantité de fluide dans le deuxième réservoir.

2. Appareil selon la revendication 1, dans lequel le dispositif de restriction est opérable pour contracter ladite partie du tissu pénien du patient ou le prolongement de celui-ci, lorsque le patient désire avoir une érection, de sorte que le flux sanguin artériel du pénis soit au moins sensiblement non restreint, tandis que le flux sanguin veineux du pénis est sensiblement restreint.

3. Appareil selon la revendication 1 ou 2, dans lequel le dispositif de restriction est conçu pour s'engager à la fois avec le corpora cavernosa ou les crura du pénis ou les prolongements de ceux-ci en tant qu'unité unique, ou pour s'engager avec chacun des deux corpora cavernosa ou crura ou le prolongement de ceux-ci en tant que deux unités séparées.

4. Appareil selon la revendication 1 ou 2, dans lequel ladite partie du tissu pénien comprend une ou plusieurs des veines de sortie du pénis.

5. Appareil selon l'une quelconque des revendications 1 à 4, dans lequel le dispositif de réglage (12 ; 52 ; 66 ; 90, 92 ; 104 ; 110 ; 436) est adapté au réglage du dispositif de restriction de manière non magnétique ou non thermique.

6. Appareil selon l'une quelconque des revendications 1 à 5, dans lequel le dispositif de réglage dispose d'une alimentation.

7. Appareil selon l'une quelconque des revendications 1 à 6, dans lequel le dispositif de restriction est non gonflable.

8. Appareil selon l'une quelconque des revendications 1 à 7, dans lequel le dispositif de restriction comprend un élément devant être placé d'un côté de ladite partie du tissu pénien, et le dispositif de réglage est adapté pour comprimer ladite partie du tissu pénien entre l'élément et l'os ou le tissu humain afin de diminuer le flux sanguin quittant le pénis.

9. Appareil selon l'une quelconque des revendications 1 à 7, dans lequel le dispositif de restriction comprend au moins un élément de restriction allongé (2 ; 48 ; 60, 62 ; 88) et un moyen de formation (10 ; 94 ; 106 ; 108 ; 118 ; 132) pour former le membre de restriction dans au moins une boucle substantiellement fermée autour de ladite partie du tissu pénien, la boucle définissant une ouverture de restriction, grâce à laquelle le dispositif de réglage est adapté au réglage du membre de restriction dans la boucle afin de modifier la taille de l'ouverture de restriction.

10. Appareil selon la revendication 9, dans lequel le dispositif de restriction comprend plusieurs membres de restriction allongés (2 ; 48 ; 60, 62 ; 88) devant être formés dans des boucles au moins substantiellement fermées autour du tissu pénien.

11. Appareil selon la revendication 9 ou 10, dans lequel le dispositif de réglage (12 ; 52 ; 66 ; 90, 92) est adapté au réglage de l'extension longitudinale du membre de restriction allongé dans ladite boucle afin de modifier la dimension de l'ouverture de la restriction.

12. Appareil selon la revendication 9, dans lequel le membre de restriction (88 ; 110 ; 122) forme une surface de confinement circonférentielle interne radiale dans ladite boucle du membre de restriction, et le dispositif de réglage (104 ; 112) est adapté au réglage mécanique du membre de restriction de sorte qu'au moins une partie de la surface de confinement soit déplacée de manière sensiblement radiale dans ladite boucle.

13. Appareil selon la revendication 12, dans lequel le membre de restriction comprend un élément annulaire élastique (110) formant la surface de confinement, et le dispositif de réglage (112) est adapté pour modifier le diamètre de l'élément annulaire élastique.

14. Appareil selon la revendication 9, dans lequel le moyen de formation (10 ; 94 ; 106 ; 108 ; 118 ; 132) est adapté pour former le membre de restriction (2 ; 48 ; 60, 62 ; 88 ; 110 ; 122 ; 126 ; 128 ; 130) dans une boucle ayant une dimension prédéterminée ou une dimension sélectionnée parmi plusieurs dimensions prédéterminées.

15. Appareil selon la revendication 9, dans lequel le dispositif de réglage (12 ; 52 ; 66 ; 90 ; 104 ; 110) est adapté pour modifier la dimension de l'ouverture de restriction (3) de sorte que la surface de confinement circonférentielle externe du membre de restriction (2 ; 48; 60, 62 ; 88 ; 110 ; 122 ; 126 ; 128 ; 130) soit modifiée.

16. Appareil selon la revendication 9, dans lequel le dispositif de réglage (12 ; 52 ; 66 ; 90 ; 104 ; 110 ; 434) est adapté pour modifier la taille de l'ouverture de restriction (3) de sorte que la surface de confinement circonférentielle externe du membre de restriction (2 ; 48; 60, 62 ; 88 ; 110 ; 122 ; 126 ; 128 ; 130 ; 436) ne soit pas modifiée.

17. Appareil selon l'une quelconque des revendications 1 à 7, dans lequel le dispositif de restriction (412) comprend au moins deux éléments (414) devant être placés sur différents côtés de ladite partie de tissu pénien (406), et le dispositif de réglage est adapté pour comprimer ladite partie du tissu pénien (406) entre les éléments afin de restreindre le flux sanguin quittant le pénis, et afin de libérer ladite partie de tissu pénien (406) des éléments afin d'augmenter le flux sanguin quittant le pénis (Figures 37A, 37B).

18. Appareil selon l'une quelconque des revendications 1 à 7 dans lequel le dispositif de restriction (418) comprend au moins deux éléments de serrage articulés (420) devant être positionnés sur des côtés opposés ou différents de ladite partie du tissu pénien (406), et le dispositif de réglage (422) est adapté pour faire tourner les éléments de serrage l'un vers l'autre afin de serrer ladite partie du tissu pénien (406) entre les éléments de serrage afin de restreindre le flux sanguin quittant le pénis, et pour faire tourner les éléments de serrage pour les desserrer l'un de l'autre afin de libérer ladite partie du tissu pénien (406) des éléments de serrage afin d'augmenter le flux sanguin quittant le pénis (Figure 38).

19. Appareil selon l'une quelconque des revendications 1 à 7, dans lequel le dispositif de restriction est adapté pour tordre une partie de ladite partie du tissu pénien (406) (Figures 39A à 44B).

20. Appareil selon la revendication 19, dans lequel le dispositif de restriction (424) comprend au moins deux membres de flexion (426-430) devant être positionnés sur des côtés opposés ou différents de ladite partie du tissu pénien (406) et devant être déplacés l'un par rapport à l'autre le long du tissu pénien, et le dispositif de réglage (432) est adapté pour déplacer les membres de flexion contre ladite partie du tissu pénien (406) pour la tordre afin de restreindre le flux sanguin quittant le pénis, et pour déplacer les membres de flexion à distance de ladite partie de tissu pénien (406) pour la libérer des membres de flexion afin d'augmenter le flux sanguin quittant le pénis (Figures 39A à 39C).

21. Appareil selon la revendication 20, dans lequel les membres de flexion comprennent des rouleaux.

22. Appareil selon l'une quelconque des revendications 1 à 7, dans lequel le dispositif de restriction est adapté pour faire tourner une partie du tissu pénien.

23. Appareil selon l'une quelconque des revendications 1 à 22, comprenant en outre un dispositif de fonctionnement pour transporter le fluide entre les troisième et deuxième réservoirs.

24. Appareil selon la revendication 23, dans lequel le dispositif de fonctionnement comprend une pompe (212).

25. Appareil selon la revendication 23 ou 24, dans lequel le dispositif de fonctionnement est opéré manuellement.

26. Appareil selon la revendication 23 ou 24, dans lequel le dispositif de fonctionnement comprend un moteur, de préférence un moteur électrique.

27. Appareil selon la revendication 26, dans lequel le moteur est réversible.

28. Appareil selon la revendication 26 ou 27, comprenant un dispositif d'inversion implantable afin d'inverser le moteur.

29. Appareil selon les revendications 1 à 28, dans lequel le moyen hydraulique est dépourvu de tout clapet de non-retour.

30. Appareil selon l'une quelconque des revendications 23 à 29, dans lequel le dispositif de fonctionnement est adapté afin d'opérer le dispositif de réglage (5) en utilisant le fluide hydraulique du premier réservoir (216).

31. Appareil selon la revendication 30, dans lequel le réservoir (216) contient une quantité prédéterminée de fluide hydraulique.

32. Appareil selon la revendication 31, dans lequel le réservoir (216) comprend les parties de la première et de la seconde parois et est adapté pour fournir un déplacement relatif entre les parties de la première et de la seconde parois du réservoir, afin de modifier le volume du réservoir.

33. Appareil selon la revendication 32, dans lequel ledit déplacement relatif est réalisé en réponse à la pression dans le réservoir.

34. Appareil selon la revendication 33, comprenant en outre une alarme adaptée pour générer un signal d'alarme en réponse à la défaillance d'une durée prédéterminée au cours de laquelle la pression contrôlant dans le troisième réservoir (16) excède une valeur élevée prédéterminée.

35. Appareil selon l'une quelconque des revendications 23 à 24, dans lequel le dispositif de fonctionnement comprend un moyen magnétique, un moyen électrique ou un moyen de manipulation manuelle ou une combinaison de ceux-ci.

36. Appareil selon l'une quelconque des revendications 1 à 35, dans lequel le deuxième réservoir (214) définit une chambre contenant une servo-fluide, et comprend les parties de la première et de la seconde parois du deuxième réservoir, qui peuvent être déplacées les unes par rapport aux autres afin de modifier le volume de la chambre du deuxième réservoir.

37. Appareil selon la revendication 36, dans lequel le troisième réservoir (210) définit une chambre pour une quantité prédéterminée supplémentaire de fluide et le dispositif de fonctionnement hydraulique est adapté pour modifier le volume de la chambre et ainsi contrôler la quantité de fluide dans le second réservoir (214).

38. Appareil selon la revendication 37, dans lequel le troisième réservoir (210) comprend des parties de la première et de la seconde parois, qui peuvent être déplacées les unes par rapport aux autres afin de modifier le volume de la chambre du troisième réservoir.

39. Appareil selon la revendication 38, dans lequel le troisième réservoir (210) augmente la quantité de fluide dans le deuxième réservoir (14) en réponse à un premier déplacement prédéterminé de la partie de la première paroi du troisième réservoir par rapport à la partie de la seconde paroi du troisième réservoir et diminue la quantité de fluide dans le deuxième réservoir en réponse à une second déplacement prédéterminé de la partie de la première paroi du troisième réservoir de fluide vers la partie de la seconde paroi du troisième réservoir.

40. Appareil selon l'une quelconque des revendications 1 à 39, dans lequel le moyen hydraulique comprend un premier réservoir (216) pouvant être implanté chez le patient et contenant une quantité prédéterminée de fluide hydraulique, et un conduit fournissant une connexion fluide entre le premier réservoir et le dispositif de restriction (2), et opère en distribuant du fluide hydraulique à travers le conduit entre le premier réservoir et le dispositif de restriction, le conduit et le dispositif de réglage de restriction étant dépourvus de tout clapet de non retour afin de permettre la libre circulation du fluide hydraulique dans les deux directions dans le conduit.

41. Appareil selon la revendication 40, dans lequel le premier réservoir (216) forme une chambre de fluide avec un volume variable, et le dispositif de réglage est adapté pour distribuer le fluide de la chambre vers le dispositif de restriction (2) en diminuant le volume de la chambre et en retirant du fluide du dispositif de restriction par expansion du volume de la chambre.

42. Appareil selon l'une quelconque des revendications 1 à 41, comprenant en outre un port d'injection pouvant être implanté de manière sous-cutanée chez le patient et en communication fluide avec le troisième réservoir (210).

43. Appareil selon la revendication 42, dans lequel le port d'injection est intégré dans le troisième réservoir (210).

44. Appareil selon l'une quelconque des revendications précédentes, comprenant en outre une commande à distance sans fil (22, 326, 332-344) pour contrôler de manière non invasive le dispositif de réglage.

45. Appareil selon la revendication 44, dans lequel la commande à distance (22, 326, 332-344) comprend un émetteur et/ou un récepteur de signal séparé (332, 336) et un récepteur et/ou un émetteur de signal pouvant être implanté (334, 338), pour transmettre et/ou recevoir un signal de commande.

46. Appareil selon la revendication 45, dans lequel le récepteur de signal (334, 338) comprend une unité de commande (338) adaptée pour commander le dispositif de réglage (12 ; 52 ; 66 ; 90, 92 ; 104 ; 110) en réponse au signal de commande.

47. Appareil selon la revendication 46, comprenant en outre une unité d'alimentation implantable (336) pour fournir de l'énergie aux composants consommant de l'énergie de l'appareil devant être implanté chez le patient.

48. Appareil selon l'une quelconque des revendications 23 à 47, dans lequel le dispositif de fonctionnement comprend une pompe ou un moteur implantable.

49. Appareil selon les revendications 47 et 48, dans lequel l'unité de commande (338) est adaptée pour commander l'unité d'alimentation (336) afin d'alimenter le moteur ou la pompe en énergie en réponse au signal de commande.

50. Appareil selon la revendication 48 ou 49, dans lequel le moteur est un moteur électrique.

51. Appareil selon la revendication 50, comprenant un transmetteur d'énergie sans fil et une unité d'alimentation, dans lequel l'unité d'alimentation (326), comprenant un moyen de transfert d'énergie adapté pour transformer l'énergie de l'énergie sans fil en énergie électrique.

52. Appareil selon la revendication 47 ou 51, dans lequel l'unité d'alimentation (26) est adaptée pour transformer l'énergie de l'énergie sans fil en un courant continu ou alternatif.

53. Appareil selon la revendication 51, dans lequel l'unité d'alimentation (326) comprend une alimentation électrique rechargeable (58) pour stocker l'énergie électrique et l'unité de commande (338) est adaptée pour alimenter le moteur électrique en énergie à partir de l'alimentation électrique rechargeable en réponse au signal de commande.

54. Appareil selon la revendication 47, dans lequel l'unité d'alimentation (326) comprend une batterie, un commutateur opérable électriquement pour connecter la batterie au récepteur de signal (334, 338) dans un mode « on » lorsque le commutateur est alimenté et pour maintenir la batterie déconnectée du récepteur de signal dans un mode « standby » lorsque le commutateur n'est pas alimenté, et une alimentation électrique rechargeable pour alimenter le commutateur.

55. Appareil selon la revendication 54, dans lequel l'unité de commande (338) est adaptée pour alimenter la pompe ou le moteur électrique en énergie de la batterie en réponse à un signal de commande reçu de l'émetteur de signal (332, 336), lorsque le commutateur est dans son mode « on ».

56. Appareil selon la revendication 47, comprenant en outre un émetteur d'énergie externe pour transmettre l'énergie sans fil, dans lequel l'unité d'alimentation comprend une batterie et un commutateur opérable par l'énergie sans fil transmise par l'émetteur externe, pour connecter la batterie au récepteur de signal dans un mode « on » lorsque le commutateur est alimenté par l'énergie sans fil et pour maintenir la batterie déconnectée du récepteur de signal dans un mode « standby » lorsque le commutateur n'est pas alimenté.

57. Appareil selon la revendication 1, comprenant en outre une unité d'alimentation pouvant être implantée (336) pour fournir de l'énergie aux composants consommant de l'énergie de l'appareil devant être implanté chez le patient.

58. Appareil selon la revendication 57, comprenant en outre un émetteur d'énergie externe pour transmettre l'énergie sans fil, dans lequel l'unité d'alimentation est adaptée pour transformer l'énergie sans fil en énergie électrique.

59. Appareil selon les revendications 26 et 58, dans lequel l'unité d'alimentation (326) est adaptée pour alimenter la pompe ou le moteur électrique en énergie électrique transformée à partir de l'énergie sans fil.

60. Appareil selon la revendication 57, comprenant en outre un émetteur d'énergie externe pour transmettre l'énergie sans fil, dans lequel l'unité d'alimentation comprend une batterie et un commutateur opérable par l'énergie sans fil transmise par l'émetteur externe, pour connecter la batterie aux composant consommant de l'énergie implantables de l'appareil dans un mode « on » lorsque le commutateur est alimenté par l'énergie sans fil et pour maintenir la batterie déconnectée des composants consommant de l'énergie dans un mode « standby » lorsque le commutateur n'est pas alimenté.

61. Appareil selon la revendication 56 ou 60, dans lequel l'émetteur d'énergie externe est adapté pour alimenter directement le commutateur en énergie sans fil afin de commuter en mode « on ».

62. Appareil selon la revendication 44, dans lequel la commande à distance (22, 326, 332-344) est capable d'obtenir des informations des composants implantables de l'appareil et de commander le dispositif de réglage (12 ; 52 ; 66 ; 90 ; 104 ; 110) pour régler le dispositif de restriction (2 ; 48 ; 60, 62 ; 88 ; 110; 122; 126; 128; 130; 434) en réponse aux informations obtenues.

63. Appareil selon la revendication 1, comprenant en outre des composants électriques implantables comprenant au moins dispositif de sécurité du niveau de tension.

64. Appareil selon la revendication 1, comprenant en outre des composants électriques implantables comprenant un seul dispositif de sécurité du niveau de tension.

65. Appareil selon la revendication 63 ou 64, dans lequel les composants électriques sont dépourvus de tout détecteur de courant et/ou détecteur de niveau de charge.

66. Appareil selon l'une quelconque des revendications 63 à 65, comprenant en outre un condensateur ou un accumulateur implantable, dans lequel la charge ou la décharge du condensateur ou de l'accumulateur est contrôlée par l'utilisation du dispositif de sécurité du niveau de tension.

67. Appareil selon la revendication 1, comprenant en outre un émetteur d'énergie sans fil et un moyen de transfert d'énergie (22, 326, 332-344) pour le transfert sans fil d'énergie sans fil de l'extérieur du corps du patient vers le dispositif de réglage et/ou d'autres composants implantables consommant de l'énergie de l'appareil

68. Appareil selon la revendication 67, dans lequel l'émetteur d'énergie est adapté pour transférer de manière intermittente l'énergie sous la forme d'un train d'impulsions d'énergie et dans lequel le moyen de transfert d'énergie transfère l'énergie sans fil en énergie électrique pour une utilisation directe en connexion avec l'alimentation des composants consommant de l'énergie de l'appareil.

69. Appareil selon la revendication 68, dans lequel le moyen de transfert d'énergie est adapté pour transférer l'énergie électrique vers un train d'impulsions électriques, et comprenant en outre un condensateur implantable pour produire le train d'impulsions d'énergie.

70. Appareil selon la revendication 66 ou 69, dans lequel le condensateur a une capacité inférieure à 0,1 µF.

71. Appareil selon la revendication 67 en association avec l'une quelconque des revendications 24 ou 26, dans lequel le moyen de transfert d'énergie est adapté pour alimenter directement le moteur ou la pompe en énergie transférée.

72. Appareil selon la revendication 24 ou 42, dans lequel le dispositif de transmission de l'énergie est adapté pour transmettre l'énergie sans fil sous la forme d'un champ magnétique ou d'ondes électromagnétiques pour l'alimentation directe du moteur ou de la pompe, pendant que l'énergie sans fil est transmise.

73. Appareil selon la revendication 72, dans lequel la pompe n'est pas une pompe de type plongeur.

74. Appareil selon la revendication 73, dans lequel le moyen de transfert d'énergie est adapté pour transférer l'énergie sans fil sous la forme d'ondes électromagnétiques à l'exclusion des ondes radio.

75. Appareil selon les revendications 67 ou 68, dans lequel l'énergie transférée par le moyen de transfert d'énergie comprend un champ électrique ou un champ magnétique.

76. Appareil selon les revendications 67 ou 68, dans lequel l'énergie transférée par le moyen de transfert d'énergie comprend un signal.

77. Appareil selon la revendication 45 ou 76, dans lequel le signal comprend des impulsions analogiques ou numériques.

78. Appareil selon la revendication 77, dans lequel les impulsions analogiques ou numériques comprennent un champ magnétique ou un champ électrique.

79. Appareil selon la revendication 45, 75 ou 76, dans lequel le signal comprend un signal d'onde.

80. Appareil selon la revendication 79, dans lequel le signal d'onde comprend un signal d'onde électromagnétique, un signal d'onde sonore ou un signal d'onde porteur.

81. Appareil selon l'une quelconque des revendications précédentes, comprenant en outre un capteur de pression pour capter directement ou indirectement la pression contre le dispositif de restriction.

82. Appareil selon la revendication 81, dans lequel le dispositif de restriction est commandé en réponse aux signaux du capteur de pression.

83. Appareil selon la revendication 1, comprenant un émetteur d'énergie sans fil et comprenant en outre un moyen de transfert d'énergie implantable pour transférer l'énergie sans fil directement ou indirectement en énergie différente de l'énergie sans fil pour le fonctionnement du dispositif de restriction.

84. Appareil selon la revendication 83, dans lequel le moyen de transfert de l'énergie transforme l'énergie sans fil en énergie cinétique pour le fonctionnement du dispositif de restriction.

85. Appareil selon la revendication 84, dans lequel le moyen de transfert de l'énergie transforme l'énergie sans fil sous la forme d'ondes sonores en énergie électrique pour le fonctionnement du dispositif de restriction.

86. Appareil selon l'une quelconque des revendications précédentes, comprenant en outre un dispositif d'inversion implantable, dans lequel le dispositif de restriction est capable de réaliser une fonction réversible et le dispositif d'inversion inverse la fonction réversible.

87. Appareil selon l'une quelconque des revendications précédentes, comprenant en outre un accumulateur ou une batterie implantable et du moyen pour commander l'accumulateur ou la batterie de l'extérieur du corps du patient pour fournir de l'énergie au dispositif de réglage et/ou aux autres composants implantables consommant de l'énergie de l'appareil.

88. Appareil selon la revendication 1, dans lequel le dispositif de réglage est adapté pour régler le dispositif de restriction de façon non manuelle.

89. Appareil selon l'une quelconque des revendications précédentes, dans lequel le dispositif de restriction est conçu pour une implantation à la base du pénis du patient ou de son prolongement.

90. Appareil selon la revendication 1, comprenant en outre un dispositif de réglage pour régler le dispositif de restriction afin de modifier la restriction du passage du flux sanguin, dans lequel le dispositif de réglage est adapté pour régler mécaniquement le dispositif de restriction, ou adapté pour régler de manière hydraulique le dispositif de restriction en utilisant un moyen hydraulique qui est dépourvu de fluide hydraulique du type ayant une viscosité qui augmente substantiellement lorsqu'il est exposé à la chaleur ou à un champ magnétique.

91. Appareil selon la revendication 90, dans lequel le dispositif de transformation de l'énergie transforme l'énergie sans fil sous la forme d'ondes sonores directement en énergie électrique.

92. Appareil selon la revendication 67, 85 ou 91, dans lequel le moyen de transfert de l'énergie comprend un condensateur.

93. Appareil selon la revendication 92, dans lequel le condensateur est adapté pour produire des impulsions électriques à partir de l'énergie électrique transformée.

94. Appareil selon la revendication 80, dans lequel le signal porteur est à modulation de fréquence, d'amplitude ou de fréquence et d'amplitude.

95. Appareil selon la revendication 80, dans lequel le signal de commande comprend un signal d'onde comprenant un signal d'onde sonore incluant un signal d'onde ultrason, un signal d'onde électromagnétique incluant un signal de lumière infrarouge, un signal de lumière visible, un signal de lumière ultraviolette et un signal de lumière laser, un signal micro-onde, un signal d'onde radio, un signal de rayonnement de rayons x, et un signal de rayonnements gamma.

96. Appareil selon la revendication 67, dans lequel le moyen de transfert d'énergie est adapté pour transférer l'énergie magnétique, l'énergie non magnétique, l'énergie électromagnétique, l'énergie non électromagnétique, l'énergie cinétique, l'énergie non cinétique, l'énergie sonique, l'énergie non sonique, l'énergie thermique ou l'énergie non thermique.

97. Appareil selon la revendication 3 ou 4, dans lequel le dispositif de restriction est adapté pour commander la surface transversale d'un passage du flux sanguin formé par les veines de sortie du pénis du patient ou corpora/crura cavernosa.

98. Appareil selon la revendication 97, dans lequel le dispositif de restriction est opérable pour ouvrir et fermer le passage de flux sanguin.

99. Appareil selon la revendication 98, dans lequel le dispositif de restriction est adapté pour commander en continu la surface transversale du passage e flux sanguin.

100. Appareil selon la revendication 67, comprenant une unité d'alimentation (326), dans laquelle l'énergie transférée est stockée dans l'unité d'alimentation pour une utilisation postérieure pour un quelconque composant implantable consommant de l'énergie de l'appareil.

101. Appareil selon la revendication 100, dans lequel l'unité d'alimentation (326) comprend un accumulateur ou une batterie rechargeable pour fournir l'énergie à un quelconque composant implantable consommant de l'énergie de l'appareil.

102. Appareil selon les revendication 48 à 49, dans lequel l'unité d'alimentation (326) comprenant un moyen de transfert, dans lequel le moyen de transfert est adapté pour transformer l'énergie du signal de commande, tel qu'il est transmis vers un récepteur de signal (334, 338), en énergie électrique.
